(19)

**Europäisches Patentamt**
**European Patent Office**
**Office européen des brevets**

(11) **EP 3 633 411 A1**

(12) **EUROPEAN PATENT APPLICATION**
published in accordance with Art. 153(4) EPC

(43) Date of publication:
**08.04.2020 Bulletin 2020/15**

(51) Int Cl.:
***G01T 1/161*** (2006.01)

(21) Application number: **17912063.9**

(22) Date of filing: **29.05.2017**

(86) International application number:
**PCT/JP2017/019944**

(87) International publication number:
**WO 2018/220686 (06.12.2018 Gazette 2018/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Shimadzu Corporation**
**Kyoto-shi, Kyoto 604-8511 (JP)**

(72) Inventor: **KOBAYASHI, Tetsuya**
**Kyoto-shi, Kyoto 604-8511 (JP)**

(74) Representative: **Mewburn Ellis LLP**
**City Tower**
**40 Basinghall Street**
**London EC2V 5DE (GB)**

(54) **ABSORPTION COEFFICIENT IMAGE ESTIMATION METHOD, ABSORPTION COEFFICIENT IMAGE ESTIMATION PROGRAM, AND POSITRON CT DEVICE EQUIPPED WITH SAME**

(57) In the attenuation coefficient image estimation method of the present invention, an attenuation coefficient value is corrected in Step S8 ($\mu = \mu' + \alpha \times \mu_{off}$) based on a mathematical relationship in which the difference between a value of a non-quantitative image $\mu'$ in a region $\Omega$ and a known attenuation coefficient value (a value of a true attenuation coefficient image) can be approximated by $\alpha \times \mu_{off}$ obtained by multiplying an offset image $\mu_{off}$ by a coefficient $\alpha$. Therefore, in the attenuation coefficient image having the attenuation coefficient value $\mu$ corrected in Step S8 ($\mu = \mu' + \alpha \times \mu_{off}$), a systematic error becomes small. As a result, a quantitative attenuation coefficient image can be generated, which enables accurate attenuation correction of the radioactivity image.

**FIG. 3**

**Description**

**Technical Field**

The present invention relates to an attenuation coefficient image estimation method, an attenuation coefficient image estimation program, a positron CT apparatus equipped with the same, which estimate an attenuation coefficient image from measurement data of a positron CT apparatus (positron emission tomography).

**Background Art**

A positron CT apparatus, i.e., a PET (Positron Emission Tomography) apparatus, is configured to measure two $\gamma$-rays generated by positron annihilation regarded as valid signals only when they are simultaneously detected by a plurality of detectors (that is, only when they are coincidentally counted), and the tomographic image of the subject is reconstructed based on the measurement data. Specifically, a radioactive pharmaceutical containing positron emitting nuclides is administered to a subject, the 511 keV annihilation $\gamma$-rays emitted from the administered subject are detected by detectors composed of a number of detector elements (e.g., scintillators). And, when $\gamma$-rays are detected within a fixed time period with two detectors, it is regarded as being detected "coincidentally", they are counted as a pair of annihilation $\gamma$-rays, and furthermore, a straight line (LOR: Line Of Response) connecting two detectors that detected the annihilation occurrence positions is specified. The coincidence counting information detected as described above is accumulated and subjected to reconstruction processing to obtain a positron emitting nuclide image (i.e., a tomographic image).

In a positron CT (PET), in order to perform a quantitative measurement of a radioactivity concentration in a subject, various data correction processing are required. Representative correction processing include sensitivity correction, scatter correction, random correction, decay correction, dead time correction, and attenuation correction,. The present invention relates to an attenuation correction for preventing a decrease in image quantitative performance due to the attenuation of $\gamma$-rays emitted from a radioactive pharmaceutical (radioisotope). In order to perform an attenuation correction, it is necessary to estimate an attenuation coefficient image representing an attenuation coefficient distribution in a subject.

In the attenuation correction, the transmittance of $\gamma$-rays is obtained from the estimated attenuation coefficient image, and the measurement data of PET is divided by the transmittance to convert into data from which the influence of the attenuation of $\gamma$-rays has been eliminated. Alternatively, the estimated attenuation coefficient image is incorporated into the calculation formula of the image reconstruction to obtain a reconstructed image from which the influence of the attenuation of $\gamma$-rays has been eliminated.

In order to estimate the attenuation coefficient image, transmission data obtained by irradiating the positron-emitting nuclide with the external radiation source is needed. Alternatively, it is possible to estimate the attenuation coefficient image by using CT data obtained from an X-ray CT (Computed Tomography) apparatus instead of transmission data.

In recent years, there is a reconstruction algorithm that does not require such transmission data (see, for example, non-Patent Documents 1 and 2). In non-Patent Documents 1 and 2, from the measurement data of PET measuring the detection time difference (TOF: time-of-flight) information of the annihilation radiation (hereinafter referred to as "TOF-PET"), it is possible to estimate the distribution shape of the attenuation coefficient sinogram. The radioactivity image and the attenuation coefficient sinogram can be estimated simultaneously. The reconstruction algorithm in non-Patent Documents 1 and 2 is also called "simultaneous reconstruction algorithm" because it simultaneously estimates the radioactivity image and the data regarding attenuation coefficient (for example, attenuation coefficient sinogram). In particular, the simultaneous reconstruction algorithm that simultaneously estimates the radioactivity image and the attenuation coefficient sinogram is referred to as an MLACF method.

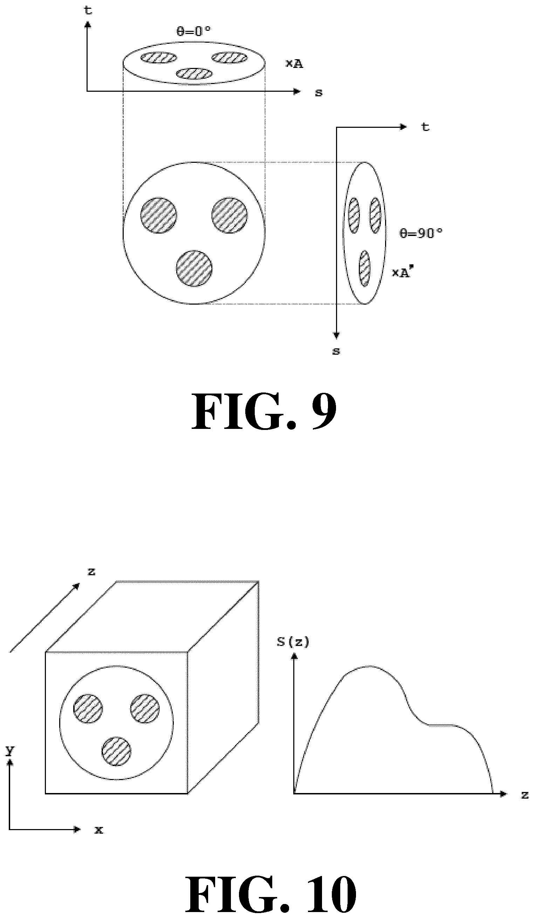

The principle of the MLACF method will be described qualitatively with reference to the conceptual diagram of FIG. 9. It is assumed that the radioactivity concentration is distributed as shown in FIG. 9 in the target region of the subject. When the radioactivity distribution is projected to a two-dimensional distribution of the TOF information t and the radial direction s, when the projection direction $\theta$ is 0°, it becomes as shown in the upper view of FIG. 9, and when the projection direction $\theta$ is 90°, it becomes as shown in the right view of FIG. 9. When the projection direction $\theta$ is in the range of 0° to 180°, a two-dimensional distribution for each projection angle $\theta$ is obtained as measurement data.

If there is no $\gamma$-ray attenuation, restoring the two-dimensional distribution per projection angle $\theta$ enables conversion into the original radioactivity distribution. However, in practice, a radioactivity distribution different from the original radioactivity distribution is restored due to $\gamma$-ray attenuation. Also, the degree of attenuation of $\gamma$-rays differs depending on the projection direction. As shown in FIG. 9, when the degree of attenuation when the projection direction $\theta$=0° is A and the degree of attenuation when the projection direction $\theta$=90° is A', usually A ≠ A'. Therefore, by minimizing the error between the measurement data and the estimated data obtained by multiplying the unknown attenuation coefficient value and the projection data, the attenuation coefficient sinogram in which the attenuation coefficient value is converted into a sinogram format can be estimated simultaneously with the radioactivity image using the measurement data of the TOF information.

However, only the distribution shape (that is, the relative value) can be estimated, and the absolute value cannot be estimated. If the absolute value of the attenuation coefficient sinogram is not known, the attenuation correction cannot be performed correctly. Therefore, a quantitative radioactivity image cannot be obtained. This is problematic in clinical situations and becomes a major barrier to practical use.

In order to put the PET imaging technology based on the simultaneous reconstruction algorithm into practical use, the following technology is indispensable. That is, a technique for estimating an attenuation coefficient image in which the absolute value is correct from an attenuation coefficient sinogram in which the distribution shape is correct but the absolute value is incorrect.

Therefore, as a conventional technique of non-Patent Document 2, there is a method of directly quantifying a radioactivity image without directly quantifying an attenuation coefficient sinogram. The processing step is as follows.

(1) a radioactivity image with no attenuation correction is generated, and a subject mask image is generated from the radioactivity image.

(2) a known attenuation coefficient (for example, an attenuation coefficient of water or an attenuation coefficient of a bone) is assigned to each pixel value of the subject mask image, and a pseudo attenuation coefficient image is generated.

(3) a radioactivity image is generated by a conventional reconstruction algorithm using a pseudo attenuation coefficient image, and a total pixel value is calculated for each slice along axial direction. As shown in FIG. 10, when z is the axial direction, the xy plane is a plane (axial plane) orthogonal to the axial direction z, and P (x, y) is the pixel value, S(z) is the total pixel value for each axial direction z slice, $S(z) = \sum y \sum x P(x, y)$. When the horizontal axis is the axial direction z and the vertical axis is a total of pixel value S(z), a profile as shown on the right of FIG. 10 is generated.

(4) a non-quantitative radioactivity image and a non-quantitative attenuation coefficient sinogram are estimated by a simultaneous reconstruction algorithm.

(5) in the same manner as in Step (3), the total pixel value is calculated for each axial direction slice of the non-quantitative radioactivity image obtained in Step (4). Here, S'(z) is the total pixel value for each axial direction z slice.

(6) the non-quantitative radioactivity image obtained in Step (4) is scaled and quantified so that the total pixel value S'(z) obtained in Step (5) and the total pixel value S(z) obtained in Step (3) coincide with each other for each axial direction slice. Specifically, the radioactivity image is scaled by multiplying each pixel value of the non-quantitative radioactivity image obtained in Step (4) by S(z)/S'(z) for each axial direction slice.

**Prior Art**

**Non-Patent Document**

Non-Patent Document 1: A. Rezaei, M. Defrise and J. Nuyts, "ML-reconstruction for TOF-PET with simultaneous estimation of the Attenuation Factors", IEEE Trans. Med. Imag., 33 (7), 1563-1572, 2014
Non-Patent Document 2: V. Y. Panin, H. Bal, M. Defrise, C. Hayden and M. E. Casey, "Transmission-less Brain TOF PET Imaging using MLACF", 2013 IEEE Nuclear Science Symposium and Medical Imaging Conference, Seoul, Republic of Korea, 2013.

**SUMMARY OF THE INVENTION**

**Problems to be Solved by the Invention**

However, in the prior art of Non-Patent Document 2 described above, there is a problem that it cannot be guaranteed in principle that the finally obtained radioactivity image is quantitative.

The present invention has been made in view of the above-described circumstances, and aims to provide an attenuation coefficient image estimation method, an attenuation coefficient image estimation program, and a positron CT apparatus equipped with the same, which are capable of generating a quantitative attenuation coefficient image.

**Means for Solving the Problems**

The inventor has obtained the following findings as a result of earnest studies to solve the above problems.

That is, the above-mentioned prior art assumes that the total pixel value for each axial direction slice obtained in the above-described Step (3) is true (that is, it is correct).

However, there is no guarantee that the total pixel value for each axial direction slice obtained in the above-described Step (3) is correct. Since the quantification is based on the result in which there is no correct guarantee, naturally, the quantitative accuracy of the radioactivity image obtained in the above-described Step (6) cannot be guaranteed. Therefore, the problem arises that the prior art cannot generate a quantitative radioactivity image. That is, the inventor has found that the root of the problem of the prior art is based on an empirical method and is not based on a mathematical theory behind the problem of quantification of the attenuation coefficient.

The present invention based on such findings has the following configuration.

That is, an attenuation coefficient image estimation method according to the present invention is an attenuation coefficient image estimation method of estimating an attenuation coefficient image from measurement data of a positron CT including time-of-flight information of annihilation radiation. The method includes:

a reconstruction calculation step of calculating an image $\mu'$ based on optimization of an evaluation function regarding the measurement data, wherein $\mu'$ is an image in which a non-uniform offset value is added to a quantitative attenuation coefficient image;

a mask calculation step of calculating subject mask projection data which is subject mask data in projection data space based on the measurement data;

an offset estimation step of estimating an offset image $\mu_{off}$ by a reconstruction algorithm configured such that forward projection data of the offset image $\mu_{off}$ approximates the subject mask projection data, where $\mu_{off}$ is a non-uniform offset image;

a reference region extraction step of extracting at least one or more regions $\Omega$ using an image in which a subject region is recognizable calculated based on the measurement data, wherein $\Omega$ is a region capable of being approximated by a known attenuation coefficient value;

a coefficient calculation step of calculating $\alpha$ which reduces an error between a value of the image $\mu'$ in the region $\Omega$ and the known attenuation coefficient value, wherein $\alpha$ is a coefficient; and

an attenuation coefficient value correction step of correcting a value obtained by adding $\alpha \times \mu_{off}$ obtained by multiplying the offset image $\mu_{off}$ by the coefficient $\alpha$ to the value of the image $\mu'$ as an attenuation coefficient value.

According to the attenuation coefficient image estimation method of the present invention, in the reconstruction calculation step, based on the optimization of the evaluation function regarding the measurement data (measurement data of TOF-PET) of the positron CT in which the time-of-flight information of the annihilation radiation is included, an image in which the non-uniform offset value is added to the quantitative attenuation coefficient image is calculated. At the same time, when $\lambda'$ is a non-quantitative radioactivity image, the radioactivity image $\lambda'$ is calculated in the reconstruction calculation step. The reconstruction algorithm in the reconstruction calculation step here is the simultaneous reconstruction algorithm described above. Here, when an image in which a non-uniform offset value is added to a quantitative attenuation coefficient image is $\mu'$, the image $\mu'$ also becomes an attenuation coefficient, but is simply referred to as an "image $\mu'$ " in distinction from the quantitative attenuation coefficient image to be finally obtained.

On the other hand, in the mask calculation step, subject mask data in the projection data space (hereinafter referred to as "subject mask projection data") is calculated based on the measurement data. When $\mu_{off}$ is a non-uniform offset image, in the offset estimation step, an offset image $\mu_{off}$ is estimated by the reconstruction algorithm configured such that the forward projection data of the offset image $\mu_{off}$ approximates the subject mask projection data.

On the other hand, when $\Omega$ is a region that can be approximated by a known attenuation coefficient value, using the image in which the subject region is recognizable calculated based on the measurement data, in the reference region extraction step, at least one region $\Omega$ is extracted.

Here, the "an image in which the subject region is recognizable calculated based on measurement data" is, for example, the image $\mu'$ mentioned above, the radioactivity image $\lambda'$ mentioned above, the non-quantitative image estimated by the reconstruction algorithm different from the reconstruction algorithm (simultaneous reconstruction algorithm) in the reconstruction calculation step mentioned above, and the subject mask image estimated from the subject mask projection data mentioned above. Of course, the image is not limited to the exemplified images, and may be any image in which the subject region is recognizable calculated based on the measurement data. Further, "extracting at least one or more regions $\Omega$ using an image in which a subject region is recognizable calculated based on the measurement data" includes not only extracting the region $\Omega$ using a single piece of image information, but also extracting the region $\Omega$ using a combination of a plurality of pieces of image information (for example, logical sum or logical product).

The following coefficient calculation step and attenuation coefficient value correction step are performed using the images $\mu'$, the offset image $\mu_{off}$, and the region $\Omega$ obtained in the above-described steps. When $\mu$ is an unknown true attenuation coefficient image value (attenuation coefficient value) and $\alpha$ is a coefficient, in the coefficient calculation step, a coefficient $\alpha$ that reduces the error between the value of the image $\mu'$ in the region $\Omega$ and a known attenuation coefficient value is calculated. In the attenuation coefficient value correction step, the value obtained by adding $\alpha \times \mu_{off}$ obtained by multiplying the offset image $\mu_{off}$ by the coefficient $\alpha$ to the value of the image $\mu'$ is corrected as an attenuation coefficient value. In this way, the attenuation coefficient value is corrected in the attenuation coefficient value correction step based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$ (i.e., $\mu \doteqdot \mu' + \alpha \times \mu_{off}$). Therefore, in the attenuation coefficient image having the attenuation coefficient value corrected in the attenuation coefficient value correction step, the systematic error becomes small. As a result, a quantitative attenuation coefficient image can be generated, which enables accurate attenuation correction of the radioactivity image.

The above-mentioned reconstruction calculation step may be performed by (a) a calculation algorithm in which the image $\mu'$ is included as an unknown variable. Alternatively, the above-mentioned reconstruction calculation step may be performed by (b) a combination of a calculation algorithm in which attenuation coefficient projection data is included as an unknown variable and an algorithm in which an image obtained by reconstructing the attenuation coefficient projection data is set as the image $\mu'$.

The former algorithm (a) is an MLAA method that simultaneously reconstructs the non-quantitative radioactivity image $\lambda'$ and the image $\mu'$ (non-quantitative attenuation coefficient image). The latter algorithm (b) is a combination of the MLACF method described in non-Patent Document 1 which simultaneously estimates the non-quantitative radioactivity image $\lambda'$ and the non-quantitative attenuation coefficient projection data (e.g., attenuation coefficient sinogram) and an algorithm in which the image obtained by reconstructing the attenuation coefficient projection data is set as the image $\mu'$. The algorithm in which the image obtained by reconstructing the attenuation coefficient projection data is set as the image $\mu'$ in the latter (b) is not specifically limited, and may be any algorithm as long as it is a reconstruction algorithm.

An example of the mask calculation step mentioned above includes a step of calculating the binarized image of the image $\mu'$ as a subject mask image, a step of calculating the projection data of the subject mask image, and a step of calculating the binarized data of the projection data of the subject mask image as a subject mask projection data (Aspect (A)). Further, the mask calculation step mentioned above includes a step of calculating the projection data of the image $\mu'$ and a step of calculating the binarized data of the projection data of the image $\mu'$ as a subject mask projection data (Aspect (B)). In the above aspect (A), the projection data is calculated after binarizing the image $\mu'$ first, and the projection data is binarized. In the above aspect (B), the projection data of the image $\mu'$ is first calculated and then binarized. The image $\mu'$ is an attenuation coefficient image that is not quantitative, but subject mask projection data can be calculated even if by binarizing the image other than the attenuation coefficient image or the projection data.

Another example of the mask calculation step includes the step of calculating binarized data obtained by converting the measurement data of TOF-PET described above into the projection data format and binarized it as subject mask projection data. In the case of this example, the data converted into the projection data format and binarized can be calculated as the subject projection data directly using the TOF-PET measurement data.

Further, another example of the mask calculation step includes a step of calculating a radioactivity image based on optimization of an evaluation function regarding the measurement data of (the above-described) TOF-PET, a step of calculating projection data of the radioactivity image, and a step of calculating data obtained by binarizing the projection data (of the radioactivity image) as the subject mask projection data (Aspect (C)). Further, the mask calculation step includes a step of calculating a radioactivity image based on optimization of an evaluation function regarding the measurement data of (the above-described) TOF-PET, a step of calculating a binarized image of the radioactivity image, a step of calculating projection data of the binarized image, and a step of calculating data obtained by binarizing projection data of the binarized image as the subject mask projection data (Aspect (D)). In the above aspect (C), the binarization is performed after the projection data of the radioactivity image is first calculated. In the above aspect (D), the projection data is calculated after binarizing the radioactivity image first, and the projection data is binarized. In any of the above aspect (C) and the above aspect (D), in the case of this example, the reconstruction algorithm for calculating the radioactivity image is not particularly limited. When the above-mentioned simultaneous reconstruction algorithm (MLACF method or MLAA method) is used, subject mask projection data can be calculated utilizing the above-mentioned radioactivity image $\lambda'$ estimated by the simultaneous reconstruction algorithm. Further, the subject mask projection data may be calculated utilizing a radioactivity image estimated by a reconstruction algorithm (for example, the ML-EM method) different from the above-mentioned simultaneous reconstruction algorithm (MLACF method or MLAA method).

The reconstruction processing performed in the above-described offset estimation step may be performed by any one of an analytical reconstruction method, a statistical reconstruction method, and an algebraic reconstruction method. For example, there is an FBP (Filtered Back Projection) method as the analytical reconstruction. As the statistical reconstruction, there is, for example, the ML-EM (Maximum Likelihood-Expectation Maximization) method described above. As the algebraic reconstruction, for example, there is the ART (Algebraic Reconstruction Technique) method.

At least one or more of regions $\Omega$ extracted in the above-described reference region extraction step is a region of a tissue in which the attenuation coefficient is regarded as known. Here, "a region of a tissue in which an attenuation coefficient is regarded as known" is, for example, a region that can be approximated by water, a region that can be approximated by air, a region that can be approximated by a brain tissue, a region that can be approximated by a bone, a region that can be approximated by a lung tissue, a region that can be approximated by a soft tissue, and the like. Of course, the region is not limited to these illustrated tissues, but may be any tissues in which an approximation value of an attenuation coefficient can be known. In the case of a brain tissue, it is regarded as a region that can be approximated by water, so the attenuation coefficient value of water of 0.0096/mm can be used.

Regarding the coefficient $\alpha$ in the above-mentioned coefficient calculation step, there are a method of calculating based on a representative value as described below (the former method) and a method of calculating based on an error evaluation function as described below (the latter method).

In the former method, K ($\geqq$ 1) is a number of regions that can be approximated by a known attenuation coefficient value, $\Omega_n$ is an $n^{th}$ region $\Omega$, $\mu^n_{known}$ (n = 1, ..., K) is the known attenuation coefficient value of the region $\Omega_n$, S(X; $\Omega_n$) is a statistic of an image X in the region $\Omega_n$ or a value calculated from the statistic as a representative value, T($x_1$, $x_2$, ..., $x_K$) is any statistic of K values $x_1$, $x_2$, ..., $x_K$ or a value calculated from statistics of K values $x_1$, $x_2$, ..., $x_K$ as a representative value, and $\alpha_n$ is the coefficient $\alpha$ in the region $\Omega_n$. Here, the representative value is represented by, for example, a mean value, a median value, a trimmed mean value, a trimmed median value, or a weighted mean value of two or more of these values. Of course, it is not limited to these exemplified values, but may be "a statistic or a value calculated from the statistic".

When the image X is replaced with the image $\mu$', S($\mu$'; $\Omega_n$) is a representative value of the image $\mu$' in the region $\Omega_n$, and when the image X is replaced with the offset image $\mu_{off}$', S($\mu_{off}$, $\Omega_n$) is a representative value of the offset image $\mu_{off}$' in the region $\Omega_n$. The known attenuation coefficient value $\mu^n_{known}$ in the region $\Omega_n$ is equal to the value obtained by adding $\alpha_n \times$S($\mu_{off}$; $\Omega_n$) obtained by multiplying the coefficient $\alpha_n$ in the region $\Omega_n$ by the representative value S($\mu_{off}$; $\Omega_n$) of the offset image $\mu_{off}$' in the region $\Omega_n$ to the representative value S($\mu$'; $\Omega_n$) of the image $\mu$' in the region $\Omega_n$. That is, the following formula is established: $\mu^n_{known}$ = S($\mu$'; $\Omega_n$) + $\alpha_n \times$S($\mu_{off}$; $\Omega_n$). Therefore, the coefficient $\alpha_n$ in the region $\Omega_n$ is calculated by $\alpha_n$ = ($\mu^n_{known}$ - S($\mu$'; $\Omega_n$)) / S($\mu_{off}$; $\Omega_n$). By replacing K pieces of values $x_1$, $x_2$, ..., $x_K$ with $\alpha_1$, $\alpha_2$, ..., $\alpha_K$, respectively, T($\alpha_1$, $\alpha_2$, ..., $\alpha_K$) is a representative value of the coefficient $\alpha_1$, $\alpha_2$, ..., $\alpha_K$. In summary, after calculating the coefficients $\alpha_1$, ..., $\alpha_K$ at each region $\Omega_1$, ..., $\Omega_K$ with n=1, ..., K, respectively, by setting the representative values T($\alpha_1$, $\alpha_2$, ..., $\alpha_K$) of the coefficients $\alpha_1$, ..., $\alpha_K$ as the coefficient $\alpha$, the coefficient $\alpha$ can be calculated.

In the latter method, K($\geqq$ 1) is a number of regions that can be approximated by a known attenuation coefficient value, $\Omega_n$ is a $n^{th}$ region $\Omega$, $\mu^n_{known}$ (n = 1, ..., K) is an image in which a known attenuation coefficient is set in the region $\Omega_n$, $D_{\Omega n}$(X, Y) is an error evaluation function of an image X and an image Y within the region $\Omega_n$, and $w_n$ (n = 1, ..., K) is a coefficient range from 0 to 1. By replacing the image X with the image $\mu^n_{known}$ in which a known attenuation coefficient is set in the region $\Omega_n$ and by replacing the image Y with the value ($\mu$' + $\alpha \times \mu_{off}$) obtained by adding $\alpha \times \mu_{off}$ obtained by multiplying the coefficient $\alpha$ by the offset image $\mu_{off}$ to the image $\mu$', $D_{\Omega n}$($\mu^n_{known}$, $\mu$'+ $\alpha \times \mu_{off}$) becomes an error evaluation function of the image $\mu^n_{known}$ in which a known attenuation coefficient is set in the region $\Omega_n$ and an image ($\mu$' + $\alpha \times \mu_{off}$) obtained by adding a non-uniform offset value to the quantitative attenuation coefficient image. By calculating $\alpha$ that minimizes the function f($\alpha$)(=$\sum_{n=1,...,K}$ [$W_n \times D_{\Omega n}$($\mu^n_{known}$, $\mu$' + $\alpha \times \mu_{off}$)]) with a coefficient $\alpha$ as a variable obtained by the weighted sum of $D_{\Omega 1}$($\mu^1_{known}$, $\mu$' + $\alpha \times \mu_{off}$) ,...., $D_{\Omega K}$($\mu^K_{known}$, $\mu$' + $\alpha \times \mu_{off}$) by the coefficients $w_1$, ..., $w_K$ for each region $\Omega_1$, ..., $\Omega_K$ at n = 1, ..., K, the coefficient $\alpha$ can be calculated.

Furthermore, the attenuation coefficient image estimation program according to the present invention makes a computer execute the attenuation coefficient image estimation method according to the present invention.

According to the attenuation coefficient image estimation program according to the invention, by making a computer execute the attenuation coefficient image estimation method according to the present invention, the attenuation coefficient value is corrected in the attenuation coefficient value correction step based on the mathematical relationship in which the difference between the value of the non-quantitative image $\mu$' in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value corrected in the attenuation coefficient value correction step, the systematic error becomes small. As a result, a quantitative attenuation coefficient image can be generated, which enables an accurate attenuation correction of the radioactivity image.

Furthermore, the positron CT apparatus according to the present invention is provided with a computing means for executing the attenuation coefficient image estimation program in the positron CT apparatus equipped with the attenuation coefficient image estimation program according to the present invention.

According to the positron CT apparatus of the present invention, the computing means configured to execute the attenuation coefficient image estimation program is provided, and the attenuation coefficient value is corrected in the attenuation coefficient value correction step based on the mathematical relationship in which the difference between the value of the non-quantitative image $\mu$' in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value corrected in the attenuation coefficient value correction step, the systematic error becomes small. As a result, a quantitative attenuation coefficient image can be generated, which enables an accurate attenuation correction of the radioactivity image.

**[Effects of the Invention]**

According to the attenuation coefficient image estimation method, the attenuation coefficient image estimation program, and the positron CT apparatus equipped with the same, the attenuation coefficient value is corrected in the attenuation coefficient value correction step based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value corrected in the attenuation coefficient value correction step, the systematic error becomes small. As a result, a quantitative attenuation coefficient image can be generated, which enables an accurate attenuation correction of the radioactivity image.

**BRIEF DESCRIPTION OF THE DRAWINGS**

FIG. 1 is a schematic perspective diagram and a block diagram of a PET apparatus according to each Example.
FIG. 2 is a schematic perspective diagram of a $\gamma$-ray detector.
FIG. 3 is a flowchart showing the processing steps and the flow of data of an attenuation coefficient image estimation method according to Example 1.
FIG. 4 is a flowchart showing the processing steps and the flow of data of an attenuation coefficient image estimation method according to Example 2.
FIG. 5 is a flowchart showing the processing steps and the flow of data of an attenuation coefficient image estimation method according to Example 3 in the case of calculating subject mask projection data using a radioactivity image estimated by the MLACF method.
FIG. 6 is a flowchart showing the processing steps and the flow of data of an attenuation coefficient image estimation method according to Example 3 in the case of calculating subject mask projection data utilizing a radioactivity image estimated by the reconstruction algorithm different from the MLACF method.
FIG. 7 is a flowchart showing the processing steps and the flow of data of an attenuation coefficient image estimation method according to Example 4.
FIG. 8 is a flowchart showing the processing steps and the flow of data of an attenuation coefficient image estimation method according to Example 5.
FIG. 9 is a conceptual diagram for explaining the principle of the MLACF method.
FIG. 10 is a schematic diagram of a total pixel value for each axial direction slice and a profile when the horizontal axis is a axial direction and the vertical axis is a total pixel value.

**[Example 1]**

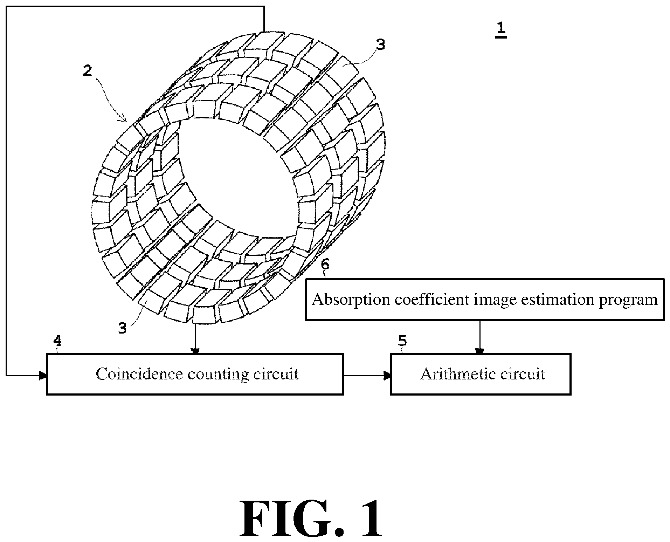

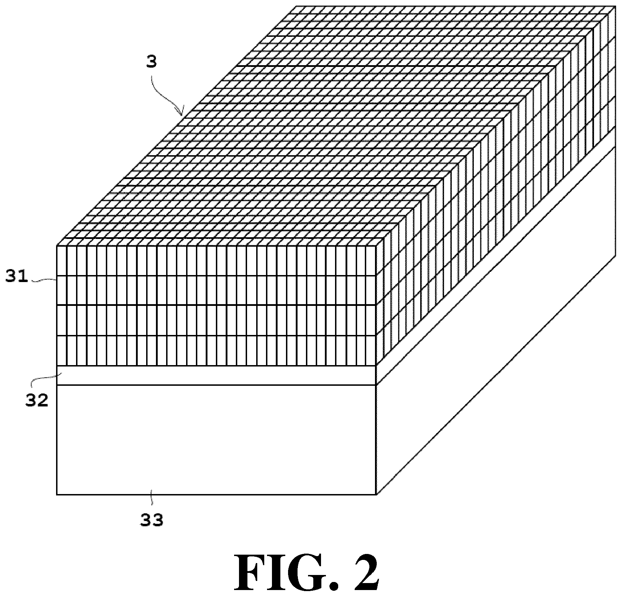

Hereinafter, Example 1 of the present invention will be described with reference to the drawings. FIG. 1 is a schematic perspective view and a block diagram of a PET apparatus according to each Example, and FIG. 2 is a schematic perspective view of a $\gamma$-ray detector. Further, FIG. 1 and FIG. 2 show a configuration common to the respective Examples.

As shown in FIG. 1, the PET apparatus 1 is provided with a detector ring 2 configured to surround a subject and stacked along the axial direction of the subject. A plurality of $\gamma$-ray detectors 3 is embedded in the detector ring 2. The PET apparatus 1 corresponds to the positron CT apparatus in the present invention. Also, the $\gamma$-ray detector 3 corresponds to the detector in the present invention.

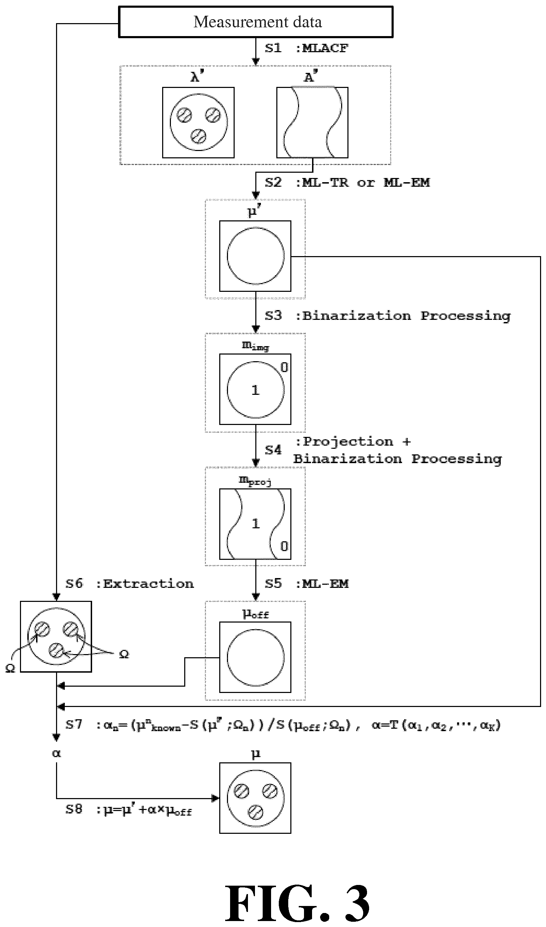

Other than the above, the PET apparatus 1 is provided with a coincidence counting circuit 4 and an arithmetic circuit 5. Although only two connections from the $\gamma$-ray detectors 3 to the coincidence counting circuit 4 are shown in FIG. 1, it should be noted that connections corresponding to the total number of channels of the photomultiplier tubes (PMT: Photo Multiplier Tube) 33 (see FIG. 2) of the $\gamma$-ray detectors 3 are connected to the coincidence counting circuit 4. The arithmetic circuit 5 executes the processing of the attenuation coefficient image estimation method shown in FIG. 3 which will be described later by the attenuation coefficient image estimation program 6. The arithmetic circuit 5 corresponds to the computing means in the present invention.

The scintillator block 31 (see FIG. 2) of the $\gamma$-ray detector 3 converts the $\gamma$-rays generated from a subject (not illustrated) to which radioactive pharmaceutical has been administered into light, and the converted light is multiplied by the photomultiplier tube (PMT) 33 (see FIG. 2) of the $\gamma$-ray detector 3 and converted into an electric signal. The electric signal is sent to the coincidence counting circuit 4 to generate detection signal data of the count value.

Specifically, when radioactive pharmaceutical is administered to a subject (not illustrated), two γ-rays are generated due to the annihilation of the positron emitted from positron emission type RI. The coincidence counting circuit 4 checks the position of the scintillator block 31 (see Fig. 2) and the incident timing of the γ-rays, and determines the sent electric signal as appropriate data only when the γ-rays are simultaneously incident on two scintillator blocks 31 on both sides of the subject. When a γ-ray is incident on only one scintillator block 31, the coincidence counting circuit 4 rejects. That is, the coincidence counting circuit 4 detects that γ-rays are simultaneously observed (that is, coincidence-counted) in two γ-ray detectors 3 based on the electric signal described above.

The detection signal data (count value) composed of appropriate data determined to be coincidence counting by the coincidence counting circuit 4 are sent to the arithmetic circuit 5. The arithmetic circuit 5 performs Steps S1 to S8 (see FIG. 3) which will be described later and estimates the attenuation coefficient image from the detection signal data of the subject (not illustrated) obtained by the PET apparatus 1. The specific functions of the arithmetic circuit 5 will be described later.

Note that the attenuation coefficient image estimation program 6 is stored in a storage medium (not illustrated) represented by a ROM (Read-only Memory), etc. The attenuation coefficient image estimation program 6 is read from the storage medium to the arithmetic circuit 5, and the attenuation coefficient image estimation program 6 is executed by the arithmetic circuit 5, thereby performing the processing of the attenuation coefficient image estimation method shown in the flowchart of FIG. 3. The arithmetic circuit 5 is configured by a programmable device (for example, an FPGA (Field Programmable Gate Array) in which the internal hardware circuit (for example, a logic circuit) can be changed according to a GPU (Graphics Processing Unit), a central processing unit (CPU), program data, and so on.

As shown in FIG. 2, the γ-ray detector 3 is provided with a scintillator block 31, a light guide 32 optically coupled to the scintillator block 31, and a photomultiplier tube (hereinafter simply abbreviated as "PMT") optically coupled to the light guide 33. Each scintillator element constituting the scintillator block 31 converts γ-rays into light by emitting light upon incidence of the γ-rays. By this conversion, the scintillator element detects the γ-rays. The light emitted in the scintillator element is sufficiently diffused in the scintillator block 31 and input to the PMT 33 through the light guide 32. The PMT 33 multiplies the light converted by the scintillator block 31 and converts it into an electric signal. The electric signal is sent to the coincidence counting circuit 4 (see FIG. 1) as a pixel value.

Further, as shown in FIG. 2, the γ-ray detector 3 is a DOI detector which is composed of scintillator elements arranged three-dimensionally and is composed of a plurality of layers arranged in the depth direction. Although FIG. 2 illustrates a four-layer DOI detector, the number of layers is not particularly limited as long as it is plural.

Here, the DOI detector is configured by laminating scintillator elements in the depth direction of radiation, and obtains the coordinate information in the depth direction (DOI: Depth of Interaction) and the lateral direction (direction in parallel to the incident surface) that caused the interaction by gravity center calculations. The spatial resolution in the depth direction can be further improved by using the DOI detector. Therefore, the number of layers of the DOI detector is the number of layers of scintillator elements stacked along the depth direction.

Next, specific functions of the arithmetic circuit 5 will be described with reference to FIG. 3. FIG. 3 is a flowchart showing the processing steps and the flow of data of the attenuation coefficient image estimation method according to Example 1.

First, a subject is scanned by the PET apparatus 1 shown in FIG. 1, and list mode data is acquired by the coincidence counting circuit 4 (see FIG. 1). In the list mode data, the energy information of the detected photon is recorded.

A standard energy window (e.g., 400 keV to 600 keV), that is, a reconstruction data energy window, and the measurement range and the bin width of the TOF measurement data in the TOF direction are set. Here, note that the bin means discretizing (dividing). In the case of an image, a pixel corresponds to a bin. The TOF bin means a time division of the TOF information. For example, when the TOF bin is 100 [ps], the detection time difference is temporally separated with accuracy of 100 [ps].

From the list mode data, the TOF-PET measurement data is generated according to the settings.

**(Step S1) MLACF**

Let μ' be an image in which an non-uniform offset value is added to the quantitative attenuation coefficient image, and let λ' be a non-quantitative radioactivity image. Based on the optimization of the evaluation function regarding the measurement data, the image μ' and the radioactivity image λ' are calculated simultaneously. As described in the "Means for Solving the Problems" section, the image μ' also becomes an attenuation coefficient image, but is simply referred to as an "image μ'" in distinction from the quantitative attenuation coefficient image to be finally obtained.

The reconstruction algorithm in Step S1 is the simultaneous reconstruction algorithm in non-Patent Document 1. In Example 1, also in Examples 2 and 3 described later, the MLACF method described in non-Patent Document 1 is applied as the simultaneous reconstruction algorithm. In regard to the specific method of the MLACF method, please refer to non-Patent Document 1 mentioned above.

Let A' be an attenuation coefficient sinogram. The radioactivity image λ' and the attenuation coefficient sinogram A' are estimated by the MLACF method. The attenuation coefficient sinogram A' corresponds to the attenuation coefficient projection data in the present invention.

**(Step S2) ML-TR or ML-EM**

The attenuation coefficient sinogram A' is reconstructed with a reconstruction algorithm (e.g., ML-TR method or ML-EM method) to generate a non-quantitative image $\mu$'. In the case of using the ML-EM method, the attenuation coefficient sinogram A' is log-transformed in advance. In regard to the specific method of the ML-TR method, please refer to Reference Document 1 (Reference Document 1: Erdoğan H, Fessler JA: Ordered subsets algorithms for transmission tomography. Phys Med Biol 44: 2835-2851, 1999). In regard to the specific method of the ML-EM method, please refer to Reference Document 2. (Reference 2: L.A. Shepp and Y. Vardi. Maximum likelihood reconstruction for emission tomography. IEEE Trans. Med. Imaging, Vol. 1, pp. 113-122, 1982). Steps S1 and S2 correspond to the reconstruction calculation step in the present invention.

**(Step S3) Binarization Processing**

The image $\mu$' is binarization-processed by threshold processing. Then, a binarized image in which the subject region is "1" and the other regions are "0" is calculated as a subject mask image. Let $m_{img}$ be a subject mask image.

**(Step S4) Projection + Binarization Processing**

The line integral data (projection data) of the subject mask image $m_{img}$ is calculated (Projection). Then, by subjecting the projection data of the subject mask image $m_{img}$ to binarization processing by threshold processing, binarized data in which the projection line passing through the subject is "1" and the other projection lines are "0" is calculated as subject mask projection data. Let $m_{proj}$ be the subject mask projection data. Steps S3 and S4 correspond to the mask calculation step in the present invention.

**(Step S5) ML-EM**

Let $\mu_{off}$ be a non-uniform offset image. The offset image $\mu_{off}$ is estimated by the reconstruction algorithm configured such that the forward projection data of the offset image $\mu_{off}$ is approximated to the subject mask projection data $m_{proj}$. That is, the subject mask projection data $m_{proj}$ is converted into image data by the reconstruction algorithm (for example, the ML-EM method). This image data is referred to as the offset image $\mu_{off}$. Step S5 corresponds to the offset estimation step in the present invention.

**(Step S6) Extraction**

Let $\Omega$ be a region that can be approximated by a known attenuation coefficient value. At least one or more regions $\Omega$ are extracted using the image in which the subject region can be recognized calculated based on the measurement data (Extraction). In Example 1, also in Examples 2 to 4 described later, when $K(\geqq 1)$ is the number of regions that can be approximated by a known attenuation coefficient value and $\Omega_n$ is the $n^{th}$ region $\Omega$, respective regions $\Omega_1$, ..., $\Omega_K$ are extracted with n = 1, ..., K. For example, in the case of scanning a head of a subject, respective regions $\Omega_1$, ..., $\Omega_K$ are extracted at a region that can be approximated by air, a region that can be approximated by a brain tissue, and a region that can be approximated by a bone.

As an image that can be recognized by the subject region calculated based on the measurement data, for example, an image $\mu$' generated in Step S2, a radioactivity image $\lambda$' obtained in Step S1, a non-quantitative image estimated by the reconstruction algorithm (e.g., ML-EM method) different from the reconstruction algorithm (simultaneous reconstruction algorithm) in Step S1, and a subject mask image $m_{img}$ estimated from the above-mentioned subject mask projection data $m_{proj}$ can be exemplified. Step S6 corresponds to the reference region extraction step in the present invention.

(Step S7) $\alpha_n = (\mu^n_{known} - S(\mu'; \Omega_n)) / S(\mu_{off}; \Omega_n)$, $\alpha = T(\alpha_1, \alpha_2, ..., \alpha_K)$

Let $\mu^n_{known}$ (n = 1, ..., K) be a known attenuation coefficient value of the region $\Omega n$, let $S(X; \Omega_n)$ be a statistic of the image X in the region $\Omega_n$ or a value calculated from the statistics as a representative value, let $T(x_1, x_2, ..., x_K)$ be any statistic of K values $x_1, x_2, ..., x_K$ or a value calculated from statistics of K values $x_1, x_2, ..., x_K$ as a representative

value , and let $\alpha_n$ be the coefficient $\alpha$ in the region $\Omega_n$. Note that $\alpha$ is a coefficient. Here, the representative value is represented by, for example, a mean value, a median value, a trimmed mean value, a trimmed median value, or a weighted mean value of two or more of these values. Here, the "trimmed mean value" means a mean value of the remaining data from which extremely large/small values are removed. Here, the "trimmed median value" means a median value of the remaining data from which extremely large/small values are removed.

When the image X is replaced with the image $\mu'$, $S(\mu'; \Omega_n)$ is a representative value of the image $\mu'$ in the region $\Omega_n$, and when the image X is replaced with the offset image $\mu_{off}$, $S(\mu_{off}; \Omega_n)$ is a representative value of the offset image $\mu_{off}$ in the region $\Omega_n$. The coefficient $\alpha_n$ in the region $\Omega_n$ is expressed by the following formula (1).

$$\alpha_n = (\mu^n_{known} - S(\mu'; \Omega_n)) / S(\mu_{off}; \Omega_n) \quad \dots (1)$$

When K pieces of values $x_1$, $x_2$, ..., $x_K$ are replaced with $\alpha_1$, $\alpha_2$, ..., $\alpha_K$, respectively, $T(\alpha_1, \alpha_2, ..., \alpha_K)$ is a representative value of the coefficient $\alpha_1$, $\alpha_2$, ..., $\alpha_K$. Therefore, the coefficient $\alpha$ is expressed by the following formula (2). Step S7 corresponds to the coefficient calculation step in the present invention.

$$\alpha = T(\alpha_1, \alpha_2, ..., \alpha_K) \quad \dots (2)$$

(Step S8) $\mu = \mu' + \alpha \times \mu_{off}$

Let $\mu$ be the value (attenuation coefficient value) of an unknown true attenuation coefficient image. The attenuation coefficient value $\mu$ is expressed by the following formula (3).

$$\mu = \mu' + \alpha \times \mu_{off} \quad \dots (3)$$

Like the above formula (3), a value obtained by adding $\alpha \times \mu_{off}$ obtained by multiplying the offset image $\mu_{off}$ by the coefficient $\alpha$ to the value of the image $\mu'$ is corrected as an attenuation coefficient value $\mu$. Step S8 corresponds to the attenuation coefficient value correction step in the present invention.

The attenuation correction is performed using the attenuation coefficient image having the attenuation coefficient value $\mu$ obtained in Step S8. As described in the "Background Art" section, the transmittance of $\gamma$-rays from the estimated attenuation coefficient image is obtained, and the transmittance is divided from the PET measurement data by converting it into the data from which the influence of the $\gamma$-ray attenuation has been eliminated to perform the attenuation correction. Alternatively, the estimated attenuation coefficient image is incorporated into the calculation formula of the image reconstruction to obtain a reconstructed image from which the influence of the attenuation of $\gamma$-rays has been eliminated to perform the attenuation correction.

According to the attenuation coefficient image estimation method of this Example 1, in Steps S1 and S2, based on the optimization of the evaluation function regarding the measurement data (measurement data of TOF-PET) of the positron CT in which the time-of-flight information of the annihilation radiation is included, an image $\mu'$ in which the non-uniform offset value is added to the quantitative attenuation coefficient image is calculated. At the same time, in Step S1, the radioactivity image $\lambda'$ is calculated. The reconstruction algorithm in Step S1 is the above-mentioned simultaneous reconstruction algorithm.

On the other hand, in Steps S3 and S4, the subject mask data (that is, subject mask projection data $m_{proj}$) in the projection data space is calculated based on measurement data. In Step S5, the offset image $\mu_{off}$ is estimated by the reconstruction algorithm configured such that the forward projection data of the offset image $\mu_{off}$ is approximated to the subject mask projection data.

On the other hand, in Step S6, at least one or more regions $\Omega$ are extracted using the image in which the subject region can be recognized calculated based on the measurement data.

Here, as described in the "Means for Solving the Problems" section, the "image in which the subject region can be recognized calculated based on the measurement data" are, for example, an image $\mu'$ generated in Step S2, a radioactivity image $\lambda'$ obtained in Step S1, a non-quantitative image estimated by the reconstruction algorithm (e.g., ML-EM method) different from the reconstruction algorithm (simultaneous reconstruction algorithm) in Step S1, and a subject mask image $m_{img}$ estimated from the above-mentioned subject mask projection data $m_{proj}$. Of course, the image is not limited to the illustrated images, and may be any image in which the subject region can be recognized calculated based on the measurement data. Further, as described in the "Means for Solving the Problems" section, "extracting at least one or more regions $\Omega$ using an image in which a subject region is recognizable calculated based on the measurement data" includes not only extracting the region $\Omega$ using a single piece of image information, but also extracting the region $\Omega$ using a combination of a plurality of pieces of image information (for example, logical sum or logical product).

Steps S7 and S8 are performed using the images $\mu'$ obtained in the above-described Steps S1 to S6, the offset image $\mu_{off}$, and the region $\Omega$. In Step S7, the coefficient $\alpha$ which reduces the error between the value of the image $\mu'$ in the region $\Omega$ and the known attenuation coefficient value is calculated. And, in Step S8, like the above-described formula (3), a value obtained by adding $\alpha \times \mu_{off}$ obtained by multiplying the offset image $\mu_{off}$ by the coefficient $\alpha$ to the value of the image $\mu'$ is corrected as an attenuation coefficient value $\mu$. In this way, the attenuation coefficient value is corrected in Step S8 based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$ (i.e., $\mu \fallingdotseq \mu' + \alpha \times \mu_{off}$). Therefore, in the attenuation coefficient image having the attenuation coefficient value $\mu$ corrected in Step S8, a systematic error becomes small. As a result, a quantitative attenuation coefficient image can be generated, which enables an accurate attenuation correction of the radioactivity image.

In Example 1, also in Examples 2 and 3 described later, Steps S1 and S2 corresponding to the above-mentioned reconstruction calculation step are performed by (b) a combination of a calculation algorithm in which an attenuation coefficient projection data is included as an unknown variable and an algorithm in which an image obtained by reconstructing the attenuation coefficient projection data is set as an image $\mu'$.

The algorithm (b) is a combination of the MLACF method described in non-Patent Document 1 which simultaneously estimates the non-quantitative radioactivity image $\lambda'$ and the non-quantitative attenuation coefficient projection data (attenuation coefficient sinogram A' in each Example 1 to 3) and an algorithm in which the image obtained by reconstructing the attenuation coefficient projection data (attenuation coefficient sinogram A') is set as the image $\mu'$. As described in the "Means for Solving the Problems" section, the algorithm in which the image obtained by reconstructing the attenuation coefficient projection data (attenuation coefficient sinogram A') is set as the image $\mu'$ in the above-described (b) is not specifically limited, and may be any algorithm as long as it is a reconstruction algorithm. In FIG. 3, as described in Step S2, the attenuation coefficient sinogram A' is reconstructed by the ML-TR method or the ML-EM method to generate an image $\mu'$.

In Example 1, Steps S3 and S4 corresponding to the above-mentioned mask calculation step are performed. That is, the mask calculation step is composed of a step of calculating a binarized image of the image $\mu'$ as a subject mask image $m_{img}$ (Step S3), a step of calculating projection data of a subject mask image $m_{img}$ ("projection" in the first half of Step S4), a step of calculating binarized data of the projection data of the subject mask image $m_{img}$ as subject mask projection data $m_{proj}$ ("Binarization Processing" in the second half of Step S4). The image $\mu'$ is an attenuation coefficient image that is not quantitative, but, as in Examples 2 and 3 described later, even if images other than the attenuation coefficient image (for example, radioactivity image $\lambda'$ or the radioactivity image $\lambda_2'$ in Example 3) and projection data are binarized, the subject mask projection data $m_{proj}$ can be calculated.

The reconstruction processing performed in Step S5 corresponding to the above-mentioned offset estimation step is performed by the statistical reconstruction calculation method represented by the ML-EM (Maximum Likelihood - Expectation Maximization) method or the like in this Example 1. Of course, the reconstruction processing performed in Step S5 is not limited to the statistical reconstruction as in this Example 1. It may be performed by any calculation method of an analytical reconstruction, a statistical reconstruction, and an algebraic reconstruction. For example, there is an FBP (Filtered Back Projection) method as the analytical reconstruction. As the algebraic reconstruction, for example, there is an ART (Algebraic Reconstruction Technique) method.

At least one or more of regions $\Omega$ extracted in Step S6 corresponding to the above-described reference region extraction step is a region of a tissue in which the attenuation coefficient is regarded as known. Here, as described in the "Means for Solving the Problems" section, the "region of a tissue in which the attenuation coefficient is regarded as known" is, for example, a region that can be approximated by water, a region that can be approximated by air, a region that can be approximated by a brain tissue, a region that can be approximated by a bone, a region that can be approximated by a lung tissue, a region that can be approximated by a soft tissue, etc. Of course, the region is not limited to these exemplified tissues, but may be any tissues in which the approximation value of the attenuation coefficient can be known. In the case of a brain tissue, it is regarded as a region that can be approximated by water, so the attenuation coefficient value of water of 0.0096/mm can be used.

In this Example 1, Step S7 corresponding to the above-mentioned coefficient calculation step is performed. That is, In Example 1, also in the Examples 2 to 4 described above, the coefficient $\alpha$ is calculated based on the representative value. As described above, the representative value is represented by, for example, a mean value, a median value, a trimmed mean value, a trimmed median value, or a weighted mean value of two or more of these values. As described in the "Means for Solving the Problems" section, it is not limited to these exemplified values, but may be "a statistic or a value calculated from the statistic".

When the image X is replaced with the image $\mu'$, $S(\mu'; \Omega_n)$ is a representative value of the image $\mu'$ in the region $\Omega_n$, and when the image X is replaced with the offset image $\mu_{off}$, $S(\mu_{off}; \Omega_n)$ is a representative value of the offset

image $\mu_{off}$' in the region $\Omega_n$. A known attenuation coefficient value $\mu^n_{known}$ in the region $\Omega_n$ is equal to the value obtained by adding $\alpha_n \times S(\mu_{off}; \Omega_n)$ obtained by multiplying the coefficient $\alpha_n$ in the region $\Omega_n$ by the representative value $S(\mu_{off}; \Omega_n)$ of the offset image $\mu_{off}$' in the region $\Omega_n$ to the representative value $S(\mu'; \Omega_n)$ of the image $\mu'$ in the region $\Omega_n$. That is, the following formula is established: $\mu^n_{known} = S(\mu'; \Omega_n) + \alpha_n \times S(\mu_{off}; \Omega_n)$. Therefore, as in the above-described formula (1), the coefficient $\alpha_n$ in the region $\Omega_n$ is calculated by $\alpha_n = (\mu^n_{known} - S(\mu'; \Omega_n)) / S(\mu_{off}; \Omega_n)$. When K pieces of values $x_1, x_2, ..., x_K$ are replaced with $\alpha_1, \alpha_2, ..., \alpha_K$, respectively, $T(\alpha_1, \alpha_2, ..., \alpha_K)$ is a representative value of the coefficient $\alpha_1, \alpha_2, ..., \alpha_K$. In summary, after calculating the coefficients $\alpha_1, ..., \alpha_K$ at each region $\Omega_1, ..., \Omega_K$ with n=1, ..., K, respectively, as in the above-described formula (2), by setting the representative values $T(\alpha_1, \alpha_2, ..., \alpha_K)$ of the coefficients $\alpha_1, ..., \alpha_K$ as the coefficient $\alpha$, the coefficient $\alpha$ can be calculated.

According to the attenuation coefficient image estimation program 6 (see FIG. 1) according to this Example 1, by making the computer (in each Example, GPU, CPU, or the programmable device constituting the arithmetic circuit 5 shown in FIG. 1) execute the attenuation coefficient image estimation program according to Example 1, the attenuation coefficient value is corrected in Step S8 based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value $\mu$ corrected in Step S8, a systematic error becomes small. As a result, a quantitative attenuation coefficient image can be generated, which enables an accurate attenuation correction of the radioactivity image.

According to the PET apparatus 1 (see FIG. 1) according to this Example 1, since the computing means (in each Example, GPU, CPU, or the programmable device constituting the arithmetic circuit 5 shown in FIG. 1 in each Example) for executing the attenuation coefficient image estimation program 6 according to Example 1 is provided, based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$, in Step S8, the attenuation coefficient is corrected. Therefore, in the attenuation coefficient image having the attenuation coefficient value $\mu$ corrected in Step S8, the systematic error becomes small. As a result, a quantitative attenuation coefficient image can be generated, which enables an accurate attenuation correction of the radioactivity image.

**[Example 2]**

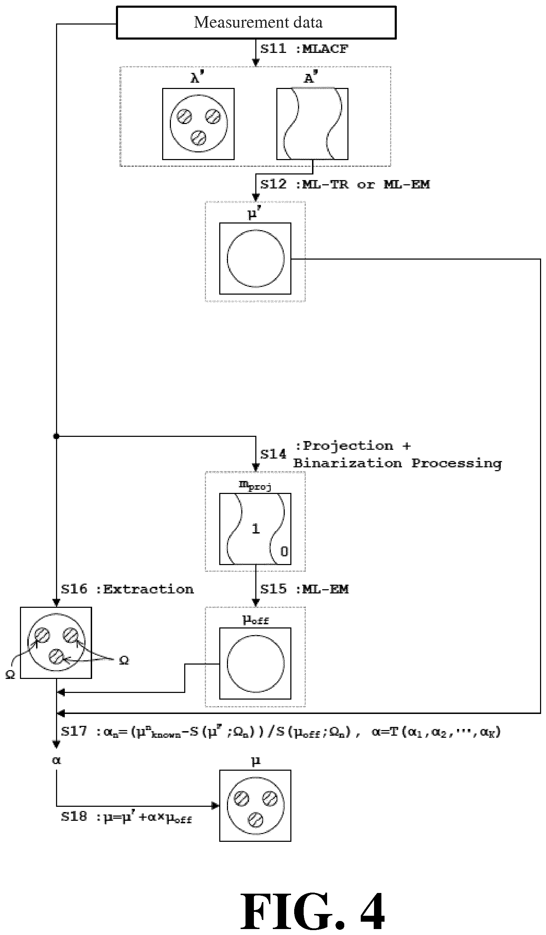

Next, Example 2 of the present invention will be described with reference to the attached drawings. FIG. 4 is a flowchart showing the processing steps and the flow of data of an attenuation coefficient image estimation method according to Example 2.

In Example 1 described above, the binarized image of the image $\mu'$ is calculated as a subject mask image $m_{img}$, the projection data of the subject mask image $m_{img}$ is calculated, and the binarized data of the projection data of the subject mask image $m_{img}$ is calculated as subject mask projection data $m_{proj}$. On the other hand, in this Example 2, the binarized data obtained by converting the measurement data of TOF-PET into a projection data format is calculated as subject mask projection data $m_{proj}$.

**(Step S11) MLACF**

Step S11 of FIG. 4 is the same as Step S1 of the above-described Example 1, and therefore the description thereof will be omitted.

**(Step S12) ML-TR or ML-EM**

Step S12 of FIG. 4 is the same as Step S2 of the above-described Example 1, and therefore the description thereof will be omitted. Steps S11 and S12 correspond to the reconstruction calculation step in the present invention.

**(Step S14) Projection + Binarization Processing**

In Example 1 described above, Steps S3 and S4 of FIG. 3 are performed to calculate the subject mask image $m_{img}$ from the image $\mu'$. In this Example 2, Step S14 of FIG. 4 is performed to calculate a subject mask image $m_{img}$ from measurement data, instead of the image $\mu'$. First, the measurement data is converted to a projection data format (Conversion). Then, by subjecting the projection data of the measurement data to binarization processing by threshold processing, binarized data in which the projection line passing through the subject is "1" and the other projection lines are "0" is calculated as subject mask projection data $m_{proj}$. Step S14 corresponds to the mask calculation step in the present invention.

**(Step S15) ML-EM**

Step S15 of FIG. 4 is the same as Step S5 of the above-described Example 1, and therefore the description thereof will be omitted. Step S15 corresponds to the offset estimation step in the present invention.

**(Step S16) Extraction**

Step S16 of FIG. 4 is the same as Step S6 of the above-described Example 1, and therefore the description thereof will be omitted. Step S16 corresponds to the reference region extraction step in the present invention.

**(Step S17) $\alpha_n = (\mu^n_{known} - S(\mu'; \Omega_n)) / S(\mu_{off}; \Omega_n)$, $\alpha = T(\alpha_1, \alpha_2, ..., \alpha_K)$**

Step S17 of FIG. 4 is the same as Step S7 of the above-described Example 1, and therefore the description thereof will be omitted. Step S17 corresponds to the coefficient calculation step in the present invention.

**(Step S18) $\mu = \mu' + \alpha \times \mu_{off}$**

Step S18 of FIG. 4 is the same as Step S8 of the above-described Example 1, and therefore the description thereof will be omitted. Step S18 corresponds to the attenuation coefficient value correction step in the present invention.

According to the attenuation coefficient image estimation method of this Example 2, in the same manner as in the above-described Example 1, the attenuation coefficient value is corrected in Step S18 based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value $\mu$ corrected in Step S18, the systematic error becomes small. As a result, a quantitative attenuation coefficient image can be generated, which enables an accurate attenuation correction of the radioactivity image.

In this Example 2, Step S14 corresponding to the above-mentioned coefficient calculation step is performed. That is, the mask calculation step includes the step of calculating binarized data obtained by converting the measurement data of TOF-PET described above into the projection data format as subject mask projection data (Step S14). In the case of this Example 2, the binarized data obtained by directly converting the measurement data of TOF-PET into a projection data format can be calculated as mask projection data $m_{proj}$.

The functions and effects of the attenuation coefficient image estimation program 6 (see FIG. 1) according to this Example 2 and the PET apparatus 1 (see FIG. 1) according to this Example 2 are the same as those in Example 1 described above, and the description will be omitted.

**[Example 3]**

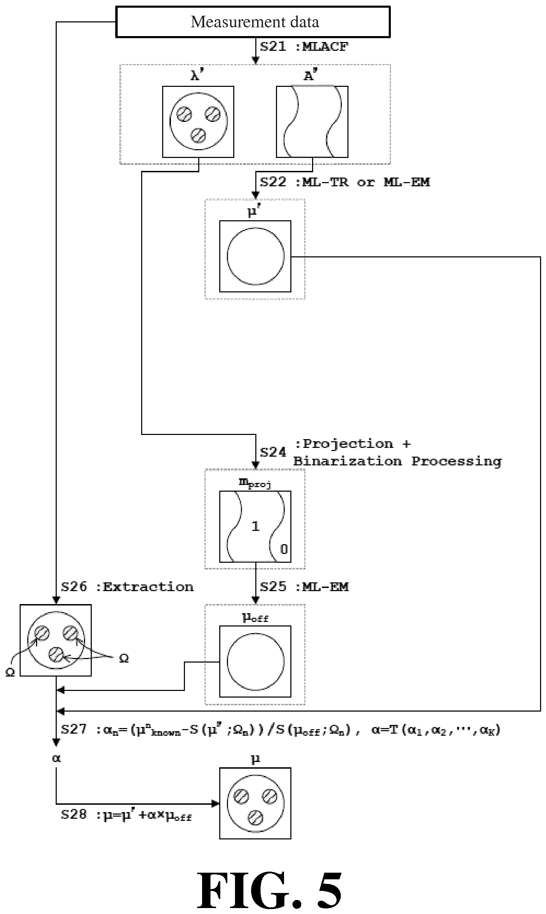

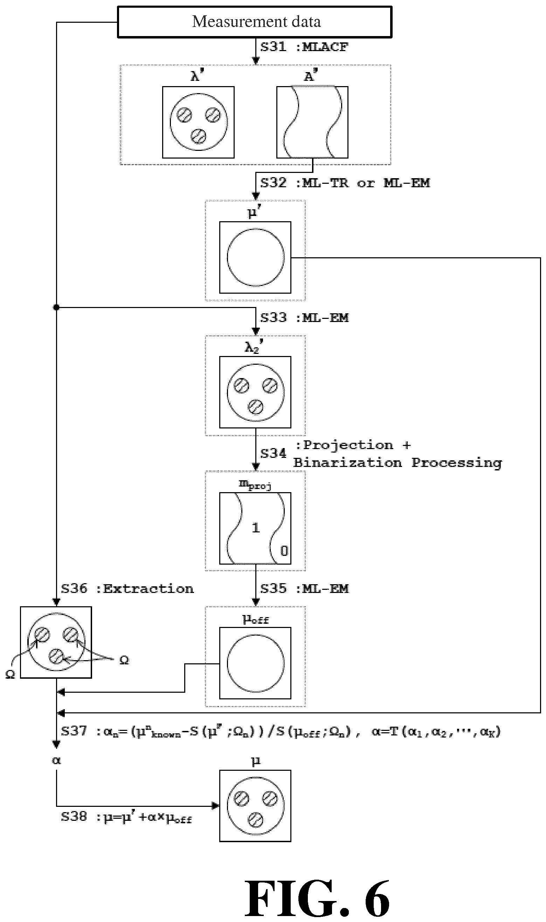

Next, Example 3 of the present invention will be described with reference to the attached drawings. FIG. 5 is a flowchart showing the processing steps and the flow of data of an attenuation coefficient image estimation method according to Example 3 in the case of calculating subject mask projection data utilizing a radioactivity image estimated by the MLACF method. FIG. 6 is a flowchart showing the processing steps and the flow of data of an attenuation coefficient image estimation method according to Example 3 in the case of calculating subject mask projection data utilizing a radioactivity image estimated by the reconstruction algorithm different from the MLACF method.

In Example 1 described above, the binarized image of the image $\mu'$ is calculated as a subject mask image $m_{img}$, the projection data of the subject mask image $m_{img}$ is calculated, and the binarized data of the projection data of the subject mask image $m_{img}$ is calculated as subject mask projection data $m_{proj}$. Further, in the above-described Example 2, the binarized data obtained by converting the measurement data of TOF-PET into a projection data format is calculated as subject mask projection data $m_{proj}$. On the other hand, in this Example 3, based on the optimization of evaluation function for measurement data of TOF-PET, the radioactivity image is calculated, the projection data of the radioactivity image is calculated, the binarized data of projection data (of radioactivity image) is calculated as subject mask projection data $m_{proj}$.

First, the case of calculating the subject mask projection data $m_{proj}$ utilizing a radioactivity image estimated by the MLACF method will be described with reference to FIG. 5.

**(Step S21) MLACF**

Step S21 of FIG. 5 is the same as Step 1 of the above-described Example 1 and Step S11 of the above-described Example 2, and therefore the description thereof will be omitted.

**(Step S22) ML-TR or ML-EM**

Step S22 of FIG. 5 is the same as Step S2 of the above-described Example 1 and Step S12 of the above-described Example 2, and therefore the description thereof will be omitted. Steps S21 and S22 correspond to the reconstruction calculation step in the present invention.

**(Step S24) Projection + Binarization Processing**

In Example 1 described above, Steps S3 and S4 of FIG. 3 are performed to calculate the subject mask image $m_{img}$ from the image $\mu'$. Further, in Example 2 described above, Step S14 of FIG. 4 is performed to calculate the subject mask image $m_{img}$ from the measurement data. In this Example 3, Step S24 of FIG. 5 is performed in order to calculate the subject mask image $m_{img}$ from the radioactivity image estimated based on the optimization of the evaluation function regarding measurement data instead of the image $\mu'$ of Example 1 and the measurement data of Example 2.

First, in FIG. 5, the line integral data (projection data) of the radioactivity image $\lambda'$ estimated based on the optimization of the evaluation function regarding the measurement data in the MLACF method is calculated (Projection). That is, in FIG. 5, the projection data of the radioactivity image $\lambda'$ may be calculated utilizing the radioactivity image $\lambda'$ estimated by the MLACF method of Step S21. Then, by subjecting the projection data of the radioactivity image $\lambda'$ to binarization processing by threshold processing, binarized data in which the projection line passing through the subject is "1" and the other projection lines are "0" is calculated as subject mask projection data $m_{proj}$. Step S24 corresponds to the mask calculation step in the present invention.

**(Step S25) ML-EM**

Step S25 of FIG. 5 is the same as Step S1 of the above-described Example 1 and Step S15 of the above-described Example 2, and therefore the description thereof will be omitted. Step S25 corresponds to the offset estimation step in the present invention.

**(Step S26) Extraction**

Step S26 of FIG. 5 is the same as Step S6 of the above-described Example 1 and Step S16 of the above-described Example 2, and therefore the description thereof will be omitted. Step S26 corresponds to the reference region extraction step in the present invention.

**(Step S27)** $\alpha_n = (\mu^n{}_{known} - S(\mu'; \Omega_n)) / S(\mu_{off}; \Omega_n), \alpha = T(\alpha_1, \alpha_2, ..., \alpha_K)$

Step S27 of FIG. 5 is the same as Step S7 of the above-described Example 1 and Step S17 of the above-described Example 2, and therefore the description thereof will be omitted. Step S27 corresponds to the coefficient calculation step in the present invention.

**(Step S28)** $\mu = \mu' + \alpha \times \mu_{off}$

Step S28 of FIG. 5 is the same as Step S8 of the above-described Example 1 and Step S18 of the above-described Example 2, and therefore the description thereof will be omitted. Step S28 corresponds to the attenuation coefficient value correction step in the present invention.

Next, the case of calculating the subject mask projection data $m_{proj}$ utilizing the radioactivity image estimated by the reconstruction algorithm different from the MLACF method will be described with reference to FIG. 6.

**(Step S31) MLACF**

Step S31 of FIG. 6 is the same as Step S21 of FIG. 5, and therefore the description thereof will be omitted.

**(Step S32) ML-TR or ML-EM**

Step S32 of FIG. 6 is the same as Step S22 of FIG. 5, and therefore the description thereof will be omitted. Steps S31 and S32 correspond to the reconstruction calculation step in the present invention.

**(Step S33) ML-EM**

In FIG. 5, Step S24 shown in FIG. 5 is performed to calculate subject mask projection data $m_{proj}$ utilizing radioactivity image $\lambda'$ estimated by the MLACF method. In FIG. 6, Step S33 and S34 of FIG. 6 are performed in order to calculate subject mask projection data $m_{proj}$ utilizing a radioactivity image estimated by a reconstruction algorithm different from the MLACF method.

First, in FIG. 6, a radioactivity image is estimated based on the optimization of the evaluation function regarding the measurement data in the reconstruction algorithm (for example, the ML-EM method) different from the MLACF method. In FIG. 6, the radioactivity image estimated by the reconstruction algorithm different from the MLACF method is set as $\lambda_2'$ in distinction from the radioactivity image $\lambda'$ estimated by the MLACF method.

**(Step S34) Projection + Binarization Processing**

The line integral data (projection data) of the radioactivity image $\lambda_2'$ estimated based on the optimization of the evaluation function regarding the measurement data in the reconstruction algorithm different from the MLACF method is calculated (Projection). Then, by subjecting the projection data of the radioactivity image $\lambda_2'$ to binarization processing by the threshold processing, binarized data in which the projection line passing through the subject is "1" and the other projection lines are "0" is calculated as subject mask projection data $m_{proj}$. Steps S33 and S34 correspond to the mask calculation step in the present invention.

**(Step S35) ML-EM**

Step S35 of FIG. 6 is the same as Step S25 of FIG. 5, and therefore the description thereof will be omitted. Step S35 corresponds to the offset estimation step in the present invention.

**(Step S36) Extraction**

Step S36 of FIG. 6 is the same as Step S26 of FIG. 5, and therefore the description thereof will be omitted. Step S36 corresponds to the reference region extraction step in the present invention.

**(Step S37)** $\alpha_n = (\mu^n{}_{known} - S(\mu'; \Omega_n)) / S(\mu_{off}; \Omega_n),\ \alpha = T(\alpha_1, \alpha_2, \dots, \alpha_K)$

Step S37 of FIG. 6 is the same as Step S27 of FIG. 5, and therefore the description thereof will be omitted. Step S37 corresponds to the coefficient calculation step in the present invention.

**(Step S38)** $\mu = \mu' + \alpha \times \mu_{off}$

Step S38 of FIG. 6 is the same as Step S28 of FIG. 5, and therefore the description thereof will be omitted. Step S38 corresponds to the attenuation coefficient value correction step in the present invention.

In the attenuation coefficient image estimation method of this Example 3, in the same manner as in the above-described Examples 1 and 2, the attenuation coefficient value is corrected in Step S28 of FIG. 5 and the attenuation coefficient value in Step S38 of FIG. 6 based on the mathematical relationship in which the difference between the value of the non-quantitative image $\mu'$ and the known attenuation coefficient value (the value of the true attenuation coefficient image) in the region $\Omega$ can be approximated by $\alpha \times \mu_{off}$ obtained by multiplying the offset image $\mu_{off}$ by the coefficient $\alpha$. Therefore, in the attenuation coefficient image having the attenuation coefficient value $\mu$ corrected in Step S28 of FIG. 5 or Step S38 of FIG. 6, the systematic error becomes small. As a result, a quantitative attenuation coefficient image can be generated, which enables an accurate attenuation correction of the radioactivity image.

In this Example 3, Step S24 of FIG. 5 corresponding to the above-mentioned coefficient calculation step or Steps S33 and S34 of FIG. 6 are performed. That is, the mask calculation step includes a step of calculating the radioactivity image ($\lambda'$ in FIG. 5 and $\lambda_2'$ in FIG. 6) based on the optimization of the evaluation function regarding the measurement data of the TOF-PET (described above) (Step S21 in FIG. 5 and Step S33 in FIG. 6), a step of calculating projection data of a radioactivity image ($\lambda'$ in FIG. 5 and $\lambda_2'$ in FIG. 6) ("Projection" in the first half of Step S24 in FIG. 5; "Projection" in the first half of Step S34 in FIG. 6), and a step of calculating data obtained by binarizing projection data (of a radioactivity image) as subject mask projection data $m_{proj}$ ("Binarization Processing" in the second half of Step S24 in FIG. 5 and "Binarization Processing" in the second half of Step S34 in FIG. 6). In the case of this Example 3, the reconstruction algorithm for calculating the radioactivity image is not particularly limited. As shown in FIG. 5, when the above-mentioned simultaneous reconstruction algorithm (MLACF method or MLAA method) is used, subject mask projection data $m_{proj}$ can be calculated utilizing the above-mentioned radioactivity image $\lambda'$ estimated by the simultaneous reconstruction algorithm. Further, as shown in FIG. 6, the subject mask projection data $m_{proj}$ may be calculated utilizing the radioactivity image $\lambda_2$' estimated by a reconstruction algorithm (for example, the ML-EM method) different from the above-mentioned simultaneous reconstruction algorithm (MLACF method or MLAA method).

The functions and effects of the attenuation coefficient image estimation program 6 (see FIG. 1) according to this Example 3 and the PET apparatus 1 (see FIG. 1) according to this Example 3 are the same as those in Examples 1 and 2 described above, and the description will be omitted.

**[Example 4]**

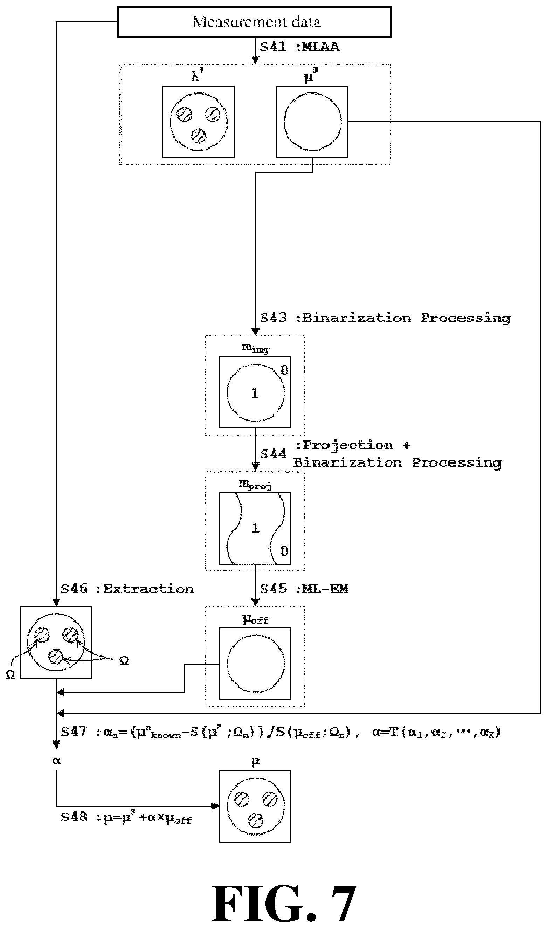

Next, Example 4 of the present invention will be described with reference to the attached drawings. FIG. 7 is a flowchart showing the processing steps and the flow of data of an attenuation coefficient image estimation method according to Example 4.

In Examples 1 to 3 as described above, the above-mentioned reconstruction calculation step is performed by the combination of the MLACF method and an algorithm in which the image obtained by reconstructing the attenuation coefficient projection data (in each of Examples 1 to 3, attenuation coefficient sinogram A') is set as the image $\mu$'. On the other hand, in this Example 4, (a) it is performed by a calculation algorithm (MLAA method) in which the image $\mu$' is included as an unknown variable.

**(Step S41) MLAA**

In Examples 1 to 3 described above, in order to calculate the image $\mu$' and the radioactivity image $\lambda$' simultaneously, after Step S1 (Step S11 in Example 2 and Step S21 or S31 in Example 3) in which the radioactivity image $\lambda$' and the attenuation coefficient sinogram A' are estimated by the MLACF method, Step S2 (Step S12 in Example 2, Steps S22 or S32 in Example 3) in which an image obtained by reconstructing the attenuation coefficient sinogram A' is set to the image $\mu$' is performed. In this Example 4, the radioactivity image $\lambda$' and the image $\mu$' are simultaneously calculated by the MLAA method.

That is, two Steps S1 and S2 (Steps S11 and S12 in Example 2, Steps S21 and S22 or S31 and S32 in Example 3) using the MLACF method are integrated into one Step S41 when the MLAA method is used in this Example 4. As for the specific method of the ML-EM method, please refer to Reference Document 3 (Reference Document 3: A Rezaei (K.U. Leuven), M. Defrise, G. Bal et. al., "Simultaneous reconstruction of activity and attenuation in time-of-flight PET", IEEE Trans. Med. Imag., 31 (12), 2224-2233, 2012). Steps S41 corresponds to the reconstruction calculation step in the present invention.

**(Step S43) Binarization Processing**

Step S43 of FIG. 7 is the same as Step S3 of the above-described Example 1, and therefore the description thereof will be omitted.

**(Step S44) Projection + Binarization Processing**

Step S44 of FIG. 7 is the same as Step S4 of the above-described Example 1, and therefore the description thereof will be omitted. Steps S43 and S44 correspond to the mask calculation step in the present invention.

**(Step S45) ML-EM**

Step S45 of FIG. 7 is the same as Step S5 of the above-described Example 1, Step S15 of the above-described Example 2, and Step of the above-described Example 3 (Step S25 in FIG. 5, Step S35 in FIG. 6), and therefore the description thereof will be omitted. Step S45 corresponds to the offset estimation step in the present invention.

**(Step S46) Extraction**

Step S46 of FIG. 7 is the same as Step S6 of the above-described Example 1, Step S16 of the above-described Example 2, and Step of the above-described Example 3 (Step S26 in FIG. 5, Step S36 in FIG. 6), and therefore the description thereof will be omitted. Step S46 corresponds to the reference region extraction step in the present invention.

**(Step S47)** $\alpha_n = (\mu^n_{known} - S(\mu'; \Omega_n)) / S(\mu_{off}; \Omega_n)$, $\alpha = T(\alpha_1, \alpha_2, ..., \alpha_K)$

Step S47 of FIG. 7 is the same as Step S7 of the above-described Example 1, Step S17 of the above-described Example 2, and Step of the above-described Example 3 (Step S27 in FIG. 5, Step S37 in FIG. 6), and therefore the description thereof will be omitted. Step S47 corresponds to the coefficient calculation step in the present invention.

**(Step S48) $\mu = \mu' + \alpha \times \mu_{off}$**

Step S48 of FIG. 7 is the same as Step S8 of the above-described Example 1, Step S18 of the above-described Example 2, and Step of the above-described Example 3 (Step S28 in FIG. 5, Step S38 in FIG. 6), and therefore the description thereof will be omitted. Step S48 corresponds to the attenuation coefficient value correction step in the present invention.

FIG. 7 is a flowchart of Example 4 when applied in cases where Steps S1 and S2 in FIG. 3 of Example 1 described above are integrated into Step S43. Of course, Example 4 may be applied in cases where Steps S11 and S12 in FIG. 4 of Example 2 described above are integrated into Step S43, and Example 4 may be applied in cases where Steps S21 and S22 in FIG. 5 of Example 3 described above or Steps S31 and S32 in FIG. 6 are integrated.

According to the attenuation coefficient image estimation method of this Example 4, in the same manner as in the above-described Examples 1 to 3, the attenuation coefficient value is corrected in Step S48 based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value $\mu$ corrected in Step S48, a systematic error becomes small. As a result, a quantitative attenuation coefficient image can be generated, which enables an accurate attenuation correction of the radioactivity image.

In this Example 4, Step S41 corresponding to the above-mentioned reconstruction calculation step may be performed by (a) a calculation algorithm in which the image $\mu'$ is included as an unknown variable. The algorithm (a) is an MLAA method that simultaneously reconstructs the non-quantitative radioactivity image $\lambda'$ and the image $\mu'$ (non-quantitative attenuation coefficient image).

The functions and effects of the attenuation coefficient image estimation program 6 (see FIG. 1) according to this Example 4 and the PET apparatus 1 (see FIG. 1) according to this Example 4 are the same as in Examples 1 and 3 described above, and the description will be omitted.

**[Example 5]**

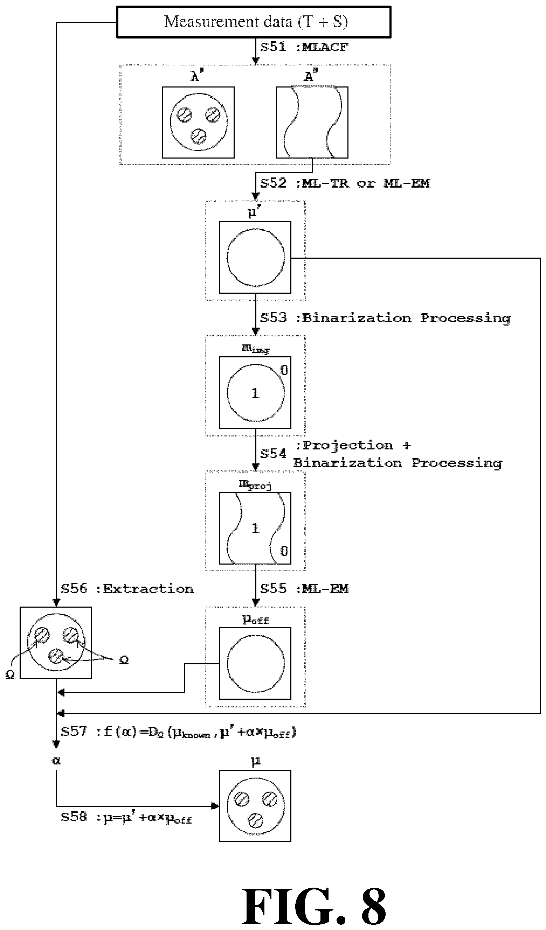

Next, Example 5 of the present invention will be described with reference to the attached drawings. FIG. 8 is a flowchart showing the processing steps and the flow of data of an attenuation coefficient image estimation method according to Example 5.

In the above-described Examples 1 to 4, the coefficient $\alpha$ is calculated based on the representative value. On the other hand, in this Example 5, the coefficient $\alpha$ is calculated based on the error evaluation function.

**(Step S51) MLACF**

Step S51 of FIG. 8 is the same as Step S1 of the above-described Example 1, Step S11 of the above-described Example 2, and Step of the above-described Example 3 (Step S21 in FIG. 5, Step S31 in FIG. 6), and therefore the description thereof will be omitted.

**(Step S52) ML-TR or ML-EM**

Step S52 of FIG. 8 is the same as Step S2 of the above-described Example 1, Step S12 of the above-described Example 2, and Step of the above-described Example 3 (Step S22 in FIG. 5, Step S32 in FIG. 6), and therefore the description thereof will be omitted. Steps S51 and S52 correspond to the reconstruction calculation step in the present invention.

**(Step S53) Binarization Processing**

Step S53 of FIG. 8 is the same as Step S3 of the above-described Example 1 and Step S43 of the above-described Example 4, and therefore the description thereof will be omitted.

**(Step S54) Projection + Binarization Processing**

Step S54 of FIG. 8 is the same as Step S4 of the above-described Example 1 and Step S44 of the above-described Example 4, and therefore the description thereof will be omitted. Steps S53 and S54 correspond to the mask calculation step in the present invention.

**(Step S55) ML-EM**

Step S55 of FIG. 8 is the same as Step S5 of the above-described Example 1, Step S15 of the above-described Example 2, Step of the above-described Example 3 (Step S25 in FIG. 5, Step S35 in FIG. 6), and Step S45 of the above-described Example 4, and therefore the description thereof will be omitted. Step S55 corresponds to the offset estimation step in the present invention.

**(Step S56) Extraction**

Step S56 of FIG. 8 is the same as Step S6 of the above-described Example 1, Step S16 of the above-described Example 2, Step of the above-described Example 3 (Step S26 in FIG. 5, Step S36 in FIG. 6), and Step S46 of the above-described Example 4, and therefore the description thereof will be omitted. Step S56 corresponds to the reference region extraction step in the present invention.

**(Step S57) $f(\alpha) = D_\Omega(\mu_{known}, \mu' + \alpha \times \mu_{off})$**

In the above-described Examples 1 to 4, the coefficient $\alpha$ is calculated based on the representative value. In this Example 5, the coefficient $\alpha$ is calculated based on the error evaluation function. When $K(\geq 1)$ is the number of regions that can be approximated by a known attenuation coefficient value and $w_n$ ($n = 1,..., K$) is a coefficient range from 0 to 1, in FIG. 8, assuming that $K = 1$ (i.e., only $n = 1$), the number of the region is only one, and coefficient $w1 = 1$, the description will be made. The generalized equation will be described later.

Specifically, let $\mu_{known}$ be an image in which a known attenuation coefficient is set in the known attenuation coefficient in the region $\Omega$, and let $D_\Omega(X, Y)$ be an error evaluation function of the image X and the image Y in the region $\Omega$. When the image X is replaced with the image $\mu_{known}$ in which a known attenuation coefficient is set in the known attenuation coefficient in the region $\Omega$ and the image Y is replaced with the value ($\mu' + \alpha \times \mu_{off}$) obtained by adding $\alpha \times \mu_{off}$ obtained by multiplying the offset image $\mu_{off}$ by the coefficient $\alpha$ to the image $\mu'$, $D_\Omega(\mu_{known}, \mu' + \alpha \times \mu_{off})$ becomes the error evaluation function of the image $\mu_{known}$ in which the known attenuation coefficient regarding the region $\Omega$ is set and the image ($\mu' + \alpha \times \mu_{off}$) obtained by adding the non-uniform offset value to the quantitative attenuation coefficient image. When $f(\alpha)$ is a function having a coefficient $\alpha$ as a variable, the function $f(\alpha)$ is expressed by the following formula (4).

$$f(\alpha) = D\Omega(\mu_{known}, \mu' + \alpha \times \mu_{off}) \quad ...(4)$$

$\alpha$ that minimizes the function $f(\alpha)$ expressed by the formula (4) is calculated. The error evaluation function $D_\Omega(X, Y)$ is, for example, a quadratic function $D_\Omega(X, Y) = \sum n_{\in \Omega}(X-Y)^2$. Of course, when it is a function that evaluates the error of the image X and the image Y, the error evaluation function $D_\Omega(X, Y)$ as exemplified in the difference sum $D_\Omega(X, Y) = \sum_{n \in \Omega}|X-Y|$ of the absolute value is not particularly limited. Step S57 corresponds to the coefficient calculation step in the present invention.

**(Step S58) $\mu = \mu' + \alpha \times \mu_{off}$**

Step S48 of FIG. 8 is the same as Step S8 of the above-described Example 1, Step S18 of the above-described Example 2, Step of the above-described Example 3 (Step S28 in FIG. 5, Step S38 in FIG. 6), and Step S48 of the above-described Example 4, and therefore the description thereof will be omitted. Step S58 corresponds to the attenuation coefficient value correction step in the present invention.

FIG. 8 is a flowchart of Example 5 applied when the calculation of coefficient $\alpha$ based on the representative value according to Step S7 in FIG. 3 of Example 1 described above is replaced with the calculation of coefficient $\alpha$ based on the error evaluation function according to Step S57. Of course, when the calculation of coefficient $\alpha$ based on the representative value according to Step S17 in FIG. 4 of Example 2 described above is replaced with the calculation of coefficient $\alpha$ based on the error evaluation function according to Step S57, Example 5 may be applied. When the calculation of coefficient $\alpha$ based on the representative value according to Step S27 in FIG. 5 of Example 3 described above is replaced with the calculation of coefficient $\alpha$ based on the error evaluation function according to Step S37 in FIG. 6, Example 5 may be applied. When the calculation of coefficient $\alpha$ based on the representative value according to Step S47 in FIG. 7 of Example 4 described above is replaced with the calculation of coefficient $\alpha$ based on the error evaluation function according to Step S57, Example 5 may be applied.

According to the attenuation coefficient image estimation method of this Example 5, in the same manner as in the above-described Examples 1 to 4, the attenuation coefficient value is corrected in Step S58 based on the mathematical relationship in which the difference between the value of the non-quantitative image $\mu'$ in the region $\Omega$ and the known attenuation coefficient value (the value of the true attenuation coefficient image) can be approximated by $\alpha \times \mu_{off}$ obtained by multiplying the offset image $\mu_{off}$ by the coefficient $\alpha$. Therefore, in the attenuation coefficient image having the attenuation coefficient value $\mu$ corrected in Step S58, a systematic error becomes small. As a result, a quantitative attenuation coefficient image can be generated, which enables an accurate attenuation correction of the radioactivity image.

In this Example 5, the coefficient $\alpha$ is calculated based on the error evaluation function. The case in which the above formula (4) is generalized will be described. Let $K (\geqq 1)$ be a region that can be approximated by a known attenuation coefficient value, let $\Omega_n$ be the $n^{th}$ region $\Omega$, let $\mu^n_{known}$ (n = 1, ..., K) be an image in which a known attenuation coefficient is set in the region $\Omega_n$, let $D_{\Omega n}(X, Y)$ be an error evaluation function of an image X and an image Y within the region $\Omega_n$, and let $w_n$ (n = 1, ..., K) be a coefficient range from 0 to 1. When the image X is replaced with the image $\mu^n_{known}$ in which a known attenuation coefficient is set in the known attenuation coefficient in the region $\Omega$ and the image Y is replaced with the value $(\mu' + \alpha \times \mu_{off})$ obtained by adding $\alpha \times \mu_{off}$ obtained by multiplying the offset image $\mu_{off}$' by the coefficient $\alpha$ to the image $\mu'$, Dnn $(\mu^n_{known}, \mu' + \alpha \times \mu_{off})$ becomes the error evaluation function of the image $\mu^n_{known}$ in which the known attenuation coefficient regarding the region $\Omega_n$ is set and the image $(\mu' + \alpha \times \mu_{off})$ obtained by adding the non-uniform offset value to the quantitative attenuation coefficient image. At this time, the function $f(\alpha)$ in which the coefficient $\alpha$ is represented by weighted sum of $D_{\Omega 1}(\mu^1_{known}, \mu' + \alpha \times \mu_{off}),..., D_{\Omega K}(\mu^K_{known}, \mu' + \alpha \times \mu_{off})$ by the coefficient $w_1,..., w_K$ for each region $\Omega_1, ..., \Omega_K$, n = 1, ... , K. That is, when generalized, the function $f(\alpha)$ is expressed by the following formula (5).

$$f(\alpha) = \Sigma_{n=1, ..., K}[w_n \times D_{\Omega n}(\mu^n_{known}, \mu' + \alpha \times \mu_{off})] \quad ...(5)$$

$\alpha$ that minimizes the function $f(\alpha)$ expressed by the formula (5) is calculated.

The functions and effects of the attenuation coefficient image estimation program 6 (see FIG. 1) according to this Example 5 and the PET apparatus 1 (see FIG. 1) according to this Example 5 are the same as in Examples 1 to 4 described above, and the description will be omitted.

The present invention is not limited to the above-described embodiment, and can be modified as follows.

(1) In each of the above-described Examples, the subject to be imaged is not particularly limited. The present invention may be applied to an apparatus for imaging an entire body of a subject, an apparatus for imaging a head of a subject, and an apparatus for imaging a breast of a subject.

(2) In each of the above-described Examples, although the DOI detector is used, the present invention may be applied to a detector that does not discriminate the depth direction.

(3) In each of the above-described Example, the detector ring is stacked along the axial direction of the subject. However, the detector ring may have only one detector ring.

(4) In each of the embodiments described above, the data format is a sinogram, but the present invention is not limited to a sinogram. As long as it is projection data, for example, the data format may be a histogram. Therefore, in Examples 1 to 3 described above, the attenuation coefficient projection data estimated by the MLACF method is the attenuation coefficient sinogram A', but the attenuation coefficient projection data estimated by the MLACF method may be the attenuation coefficient histogram.

(5) In Example 1 described above, the projection data is calculated after binarizing the image $\mu'$ first, the and projection data is binarized. However, the subject mask projection data $m_{proj}$ may be calculated as follows. That is, the mask calculation step may be an aspect including a step of calculating projection data of the image $\mu'$ and a step of calculating binarized data of the projection data of the image $\mu'$ as a subject mask projection data $m_{proj}$. In the case of this aspect, the projection data of the image $\mu'$ is first calculated and then binarized.

(6) In Example 3 described above, the projection data of the radioactivity image ($\lambda'$ in FIG. 5 and $\lambda_2'$ in FIG. 6) is calculated first and then binarized, but the subject mask projection data $m_{proj}$ is calculated as follows. That is, the mask calculation step may be an aspect including a step of calculating a radioactivity image based on optimization of an evaluation function regarding the measurement data of (the above-described) the TOP-PET, a step of calculating a binarized image of the radioactivity image, a step of calculating projection data of the binarized image, and a step of calculating data obtained by binarizing projection data of the binarized image as the subject mask projection data $m_{proj}$. In the case of the aspect, the projection data is calculated after binarizing the radioactivity image first, and the projection data is binarized. In the same manner as in Example 3 described above, also in this aspect, the reconstruction algorithm for calculating the radioactivity image is not particularly limited. A simultaneous reconstruction algorithm (MLACF method or MLAA method) as shown in FIG. 5 or a reconstruction algorithm (e.g. ML-EM method) different from the simultaneous reconstruction algorithm (MLACF method or MLAA method) as shown in FIG. 6 may be used.

**Industrial Applicability**

As described above, the present invention is suitable for estimation of an attenuation coefficient image using measurement data measured by a TOF measurement type PET apparatus.

**Description of Reference Symbols**

1 ... PET apparatus
3 ... $\gamma$-ray detector
5 ... arithmetic circuit
6 ... attenuation coefficient image estimation program
$\lambda$', $\lambda_2$' ... radioactivity image
A' ... attenuation coefficient sinogram
$\mu$' ... image (a non-uniform offset value is added to a quantitative attenuation coefficient image)
mimg ... subject mask image
$m_{proj}$ ... subject mask projection data
$\mu_{off}$ ... (non-uniform) offset image
$\Omega$ ... region (that can be approximated by a known attenuation coefficient value)
K ... number of regions that can be approximated by known attenuation coefficient values
$\Omega_n$ ... $n^{th}$ region $\Omega$
$\mu^n_{known}$ ... known attenuation coefficient value of the region $\Omega_n$
$S(\mu';\Omega_n)$ ... representative value of the image $\mu$' in the region $\Omega_n$
$S(\mu_{off}; \Omega_n)$ ... representative value of the offset image $\mu_{off}$ in the region $\Omega_n$
$\alpha$ ... coefficient
$\alpha_n$ ... coefficient $\alpha$ in the region $\Omega_n$
$T(\alpha_1, \alpha_2, ..., \alpha_K)$ ... representative value of the coefficient $\alpha_1$, $\alpha_2$, ..., $\alpha_K$
$\mu$ ... value of the true attenuation coefficient image (attenuation coefficient value)
$D_{\Omega}(\mu_{known}, \mu' + \alpha \times \mu_{off})$ ... error evaluation function of the image $\mu_{known}$ in which the known attenuation coefficient is set in the region $\Omega_n$ and the image ($\mu$' + $\alpha \times \mu_{off}$) in which the non-uniform offset value is added to the quantitative attenuation coefficient image $D_{\Omega n}(\mu^n_{known}, \mu' + \alpha \times \mu_{off})$ ... error evaluation function of the image $\mu^n_{known}$ in which the known attenuation coefficient is set in the region $\Omega_n$ and the image ($\mu$' + $\alpha \times \mu_{off}$) in which the non-uniform offset value is added to the quantitative attenuation coefficient image $w_n$ ... coefficient (range from 0 to 1)
$f(\alpha)$ ... function (coefficient $\alpha$ is variable)

**Claims**

1.  An attenuation coefficient image estimation method of estimating an attenuation coefficient image from measurement data of a positron CT including time-of-flight information of annihilation radiation, the method comprising:

    a reconstruction calculation step of calculating an image $\mu$' based on optimization of an evaluation function regarding the measurement data, wherein $\mu$' is an image in which a non-uniform offset value is added to a quantitative attenuation coefficient image;
    a mask calculation step of calculating subject mask projection data which is subject mask data in projection data space based on the measurement data;
    an offset estimation step of estimating an offset image $\mu_{off}$ by a reconstruction algorithm configured such that forward projection data of the offset image $\mu_{off}$ approximates the subject mask projection data, where $\mu_{off}$ is a non-uniform offset image;
    a reference region extraction step of extracting at least one or more regions $\Omega$ using an image in which a subject region is recognizable calculated based on the measurement data, wherein $\Omega$ is a region capable of being approximated by a known attenuation coefficient value;

a coefficient calculation step of calculating a coefficient $\alpha$ which reduces an error between a value of the image $\mu'$ in the region $\Omega$ and the known attenuation coefficient value, wherein $\alpha$ is a coefficient; and
an attenuation coefficient value correction step of correcting a value by adding $\alpha \times \mu_{off}$ obtained by multiplying the offset image $\mu_{off}$ by the coefficient $\alpha$ to the value of the image $\mu'$ as an attenuation coefficient value.

2.  The attenuation coefficient image estimation method as recited in claim 1,
    wherein the reconstruction calculation step is performed by (a) a calculation algorithm in which the image $\mu'$ is included as an unknown variable, or (b) a combination of a calculation algorithm in which attenuation coefficient projection data is included as an unknown variable and an algorithm in which an image obtained by reconstructing the attenuation coefficient projection data is set as the image $\mu'$.

3.  The attenuation coefficient image estimation method as recited in claim 1 or 2, wherein

    (A) the mask calculation step comprises:

        a step of calculating a binarized image of the image $\mu'$ as a subject mask image;
        a step of calculating projection data of the subject mask image; and
        a step of calculating binarized data of the projection data of the subject mask image as the subject mask projection data,
        or

    (B) the mask calculation step comprises:

        a step of calculating projection data of the image $\mu'$; and
        a step of calculating binarized data of the projection data of the image $\mu'$ as the subject mask projection data.

4.  The attenuation coefficient image estimation method as recited in claim 1 or 2, wherein the mask calculation step comprises a step of calculating binarized data of a data obtained by converting the measurement data into projection data format as the subject mask projection data.

5.  The attenuation coefficient image estimation method as recited in claim 1 or 2, wherein

    (C) the mask calculation step comprises
    a step of calculating a radioactivity image based on optimization of an evaluation function regarding the measurement data;
    a step of calculating projection data of the radioactivity image; and
    a step of calculating data by binarizing the projection data as the subject mask projection data,
    or
    (D) the mask calculation step comprises
    a step of calculating a radioactivity image based on optimization of an evaluation function regarding the measurement data;
    a step of calculating a binarized image of the radioactivity image;
    a step of calculating projection data of the binarized image; and
    a step of calculating data by binarizing projection data of the binarized image as the subject mask projection data.

6.  The attenuation coefficient image estimation method as recited in any one of claims 1 to 5,
    wherein reconstruction processing performed in the offset estimation step is performed by any one of an analytical reconstruction method, a statistical reconstruction method, and an algebraic reconstruction method.

7.  The attenuation coefficient image estimation method as recited in any one of claims 1 to 6,
    wherein the at least one or more regions $\Omega$ extracted in the reference region extraction step is a region of a tissue in which an attenuation coefficient is regarded as known.

8.  The attenuation coefficient image estimation method as recited in any one of claims 1 to 7,
    wherein when
    $K(\geqq 1)$ is the number of regions that can be approximated by the known attenuation coefficient value,
    $\Omega_n$ is an $n^{th}$ region $\Omega$, $\mu^n_{known}$ (n = 1, ..., K) is the known attenuation coefficient value of the region $\Omega_n$,
    $S(X; \Omega_n)$ is a statistic of an image X in the region $\Omega_n$ or a value calculated from the statistic as a representative value,

$T(x_1, x_2, ..., x_K)$ is any statistic of K values $x_1, x_2, ..., x_K$ or a value calculated from statistics of K values $x_1, x_2, ..., x_K$ as a representative value, and

$\alpha_n$ is the coefficient $\alpha$ in the region $\Omega_n$,

the coefficient $\alpha$ in the coefficient calculation step is $\alpha = T(\alpha_1, \alpha_2, ..., \alpha_k)$, $\alpha_n = (\mu^n_{known} - S(\mu'; \Omega_n))/S(\mu_{off}; \Omega_n)$ (n = 1, ..., K).

9. The attenuation coefficient image estimation method as recited in any one of claims 1 to 7, wherein when

$K(\geqq 1)$ is the number of regions that can be approximated by the known attenuation coefficient value,

$\Omega_n$ is an $n^{th}$ region $\Omega$,

$\mu^n_{known}$ (n = 1, ..., K) is an image in which a known attenuation coefficient is set in the region $\Omega_n$,

$D_{\Omega n}(X, Y)$ is an error evaluation function of an image X and an image Y within the region $\Omega n$, and

$w_n$ (n = 1, ..., K) is a coefficient range from 0 to 1,

the coefficient $\alpha$ in the coefficient calculation step is $\alpha$ which minimizes a function $f(\alpha) = \sum_{n=1,...,K}[w_n \times D_{\Omega n}(\mu^n_{known}, \mu' + \alpha \times \mu_{off})]$.

10. An attenuation coefficient image estimation program that makes a computer execute the attenuation coefficient image estimation method as recited in any one of claims 1 to 9.

11. A positron CT apparatus equipped with the attenuation coefficient image estimation program as recited in claim 10, comprising:

a computing means configured to execute the attenuation coefficient image estimation program.

**FIG. 1**

**FIG. 2**

**FIG. 3**

Measurement data

↓ S11 :MLACF

λ'

A'

↓ S12 :ML-TR or ML-EM

μ'

S14 :Projection +
Binarization Processing

m_proj

1

0

S16 :Extraction

S15 :ML-EM

Ω

Ω

μ_off

S17 : $\alpha_n = (\mu^n_{known} - S(\mu';\Omega_n))/S(\mu_{off};\Omega_n)$, $\alpha = T(\alpha_1, \alpha_2, \cdots, \alpha_K)$

α

μ

S18 : $\mu = \mu' + \alpha \times \mu_{off}$

# FIG. 4

**FIG. 5**

**FIG. 6**

FIG. 7

**FIG. 8**

# FIG. 9

# FIG. 10

## INTERNATIONAL SEARCH REPORT

International application No.

PCT/JP2017/019944

| A. | CLASSIFICATION OF SUBJECT MATTER |
|---|---|

*G01T1/161*(2006.01)i

According to International Patent Classification (IPC) or to both national classification and IPC

| B. | FIELDS SEARCHED |
|---|---|

Minimum documentation searched (classification system followed by classification symbols)
G01T1/161-1/166

Documentation searched other than minimum documentation to the extent that such documents are included in the fields searched

| Jitsuyo Shinan Koho | 1922-1996 | Jitsuyo Shinan Toroku Koho | 1996-2017 |
|---|---|---|---|
| Kokai Jitsuyo Shinan Koho | 1971-2017 | Toroku Jitsuyo Shinan Koho | 1994-2017 |

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | LI, Yusheng et al., Transmission-less attenuation estimation from time-of-flight PET histo-images using consistensy equations, Physics in Medicine and Biology, 2015.08.21, Volume 60, Number 16, 6563 | 1-11 |
| A | LI, Quanzheng et al., Joint estimation of activity image and attenuation sinogram using time-of-flight positron emission tomography data consistency condition filtering, Journal of Medical Imaging, 2017.04.26, Volume 4, Issue 2, 023502 | 1-11 |
| A | US 2015/0193927 A1 (WANG, Ge), 09 July 2015 (09.07.2015), entire text; all drawings (Family: none) | 1-11 |

| ☒ Further documents are listed in the continuation of Box C. | ☐ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 09 August 2017 (09.08.17) | 22 August 2017 (22.08.17) |

| Name and mailing address of the ISA/ | Authorized officer |
|---|---|
| Japan Patent Office 3-4-3,Kasumigaseki,Chiyoda-ku, Tokyo 100-8915,Japan | Telephone No. |

Form PCT/ISA/210 (second sheet) (January 2015)

## INTERNATIONAL SEARCH REPORT

International application No.

PCT/JP2017/019944

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2015-519555 A  (Koninklijke Philips N.V.), 09 July 2015 (09.07.2015), entire text; all drawings & US 2015/0098640 A1 entire text; all drawings & WO 2013/164731 A1    & EP 2844148 A1 & CN 104271045 A      & RU 2014148796 A | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **A. REZAEI ; M. DEFRISE ; J. NUYTS.** ML-reconstruction for TOF-PET with simultaneous estimation of the Attenuation Factors. *IEEE Trans. Med. Imag.,* 2014, vol. 33 (7), 1563-1572
- Transmission-less Brain TOF PET Imaging using MLACF. **V. Y. PANIN ; H. BAL ; M. DEFRISE ; C. HAYDEN ; M. E. CASEY.** IEEE Nuclear Science Symposium and Medical Imaging Conference. Seoul, Republic of Korea, 2013

- **H, FESSLER JA.** Ordered subsets algorithms for transmission tomography. *Phys Med Biol,* 1999, vol. 44, 2835-2851
- **L.A. SHEPP ; Y. VARDI.** Maximum likelihood reconstruction for emission tomography. *IEEE Trans. Med. Imaging,* 1982, vol. 1, 113-122
- **A REZAEI ; K.U. LEUVEN ; M. DEFRISE ; G. BAL.** Simultaneous reconstruction of activity and attenuation in time-of-flight PET. *IEEE Trans. Med. Imag.,* 2012, vol. 31 (12), 2224-2233